# EUROPEAN PATENT SPECIFICATION

(11) **EP 3 526 232 B1**
(45) Date of publication and mention of the grant of the patent: **02.12.2026**
(21) Application number: 17860762.8
(22) Date of filing: 11.10.2017
(51) Int. Cl.: C07K 5/103, C07K 7/06, C07K 7/08, C12Q 1/34, C12Q 1/37, G01N 27/26, G01N 33/53

(54) **DETECTION, IDENTIFICATION, AND PURIFICATION OF DEGRADATIVE AND NON-DEGRADATIVE ENZYMES IN BIOLOGICAL SAMPLES**
NACHWEIS, IDENTIFIZIERUNG UND REINIGUNG VON ABBAUBAREN UND NICHT-ABBAUBAREN ENZYMEN IN BIOLOGISCHEN PROBEN
DÉTECTION, IDENTIFICATION ET PURIFICATION D'ENZYMES DE DÉGRADATION ET DE NON DÉGRADATION DANS DES ÉCHANTILLONS BIOLOGIQUES

(30) Priority: 11.10.2016 US 201662406781 P
(43) Date of publication of application: 21.08.2019
(73) Proprietor: The Regents of the University of California, Oakland, CA 94607-5200 (US)
(72) Inventor: HELLER, Michael J., Poway California 92064 (US); MODESTINO, Augusta E., Portland Oregon 97210 (US); SCHMID SCHONBEIN, Geert W., Del Mar California 92024 (US); SKOWRONSKI, Elaine, Encinitas California 92024 (US); LEITERER, Christian, 07749 Jena (DE)
(74) Representative: Fish & Richardson P.C.
(86) International application number: PCT/US2017/056163
(87) International publication number: WO 2018/071556

(56) References cited:
- US-A1- 2002 142 351
- US-A1- 2007 141 624
- US-A1- 2010 280 414
- US-A1- 2010 285 573
- US-A1- 2014 079 632
- US-A1- 2016 039 792
- US-A1- 2016 169 885
- EDGINGTON ET AL.: "Functional Imaging of Proteases: Recent Advances in the Design and Application of Substrate-Based and Activity-Based Probes", CURRENT OPINION IN CHEMICAL BIOLOGY, vol. 15, 31 December 2011 (2011-12-31), pages 798 - 805, XP028338677
- MODESTINO ET AL.: "Thrombin Generation Assay in Untreated Whole Human Blood", ELECTROPHORESIS, vol. 37, 9 August 2016 (2016-08-09), pages 2248 - 2256, XP055500633
- YANG ET AL.: "Modification of Hydrophilic and Hydrophobic Surfaces Using an Ionic-Complementary Peptide", PLOS ONE, vol. 2, 19 December 2007 (2007-12-19), pages 1 - 11, XP055500635

## Description

### TECHNICAL FIELD

This disclosure relates generally to methods of detecting protease activity in a complex biological sample, in particular methods of detecting degradative and non-degradative enzymatic activity in a complex biological sample, such as blood, plasma, or serum, without sample processing.

### BACKGROUND

Over 300 protein post-translational modifications have been identified, and they profoundly influence protein function, localization, activity, and structure. Common post-translational modifications include phosphorylation, glycosylation, conjugation of small proteins such as ubiquitin and SUMO (small ubiquitin-related modifier), and acetylation of the N terminus and lysine residues, but the proteolytic modification of proteins is often overlooked in its importance and ubiquity. Indeed, many biomarkers of disease are stable proteolytic fragments in biological fluids.

Proteases are enzymes that carry out the hydrolysis of peptide bonds within proteins and polypeptides. Besides their roles in digestion and protein turnover, proteases function in tightly regulated cascades and signaling events. By precise proteolytic processing, proteases participate in every biological process including DNA replication and repair, cell cycle progression, cell proliferation, differentiation and migration, morphogenesis and tissue remodeling, neuronal outgrowth, hemostasis, wound healing, immunity, angiogenesis, and apoptosis. Indeed, more than 53 specific hereditary diseases of proteolysis are recognized, and it is not surprising then that proteases are implicated in many pathologies. In addition, numerous pathogens use proteases during the invasion of their host, for their replication inside the host, or in modulating the host's immune response.

For these reasons, proteases are considered important targets for therapeutic intervention. Proteases represent 5-10% of drug targets. With six classes of proteases that account for ~2% of the genes of any organism (see the peptidase database MEROPs for useful information), proteases constitute the second largest enzyme family in man after ubiquitin ligases, totaling more than 567 proteases. Several protease inhibitors have been developed into drugs against such diseases as HIV infection, multiple myeloma, and hypertension. Despite these successes, many protease inhibitors have failed in clinical trials due to a lack of selectivity or misconceptions about the functional role of the targeted protease(s).

These examples underscore the importance of having a better understanding of protease activity in a more complex and biologically relevant setting as part of the early drug discovery process, life sciences research, and diagnostics development.

US 2014/0079632 describes selective trypsine-like serine protease activity-based probes, in particular urokinase plasminogen activator-activity based probes, the use thereof and methods for detecting selective urokinase activity by making use of said probes.

US 2007/141624 describes compositions and methods for assessing profiles of catalytically active enzymes in compositions containing a plurality of proteins. The methods described use activity based probes ("ABPs") that have an affinity moiety for directing the binding of the ABP to one or more catalytically active target enzymes, a reactive group for forming a covalent bond at an active site of the target enzyme(s), and a tag. One or more ABPs may be combined with a protein-containing sample under conditions for binding and reaction of the ABP(s) with target enzyme(s) that are present in the sample. The resulting products may then be used to assess the active enzyme profile of the sample.

US 2016/169885 describes compositions useful in detecting degradative enzymes and biomolecules in bodily fluid samples.

US 2016/039792 describes activity-based probe compounds for use in labeling a cysteine protease. The compounds are targeted to the protease through a specific targeting element. The compounds additionally include a detectable element, such as a fluorescent label, a radiolabel, or a chelator. Also described are compositions comprising the compounds and methods for using the compounds, for example in labeling a protease in an animal and in visualizing a tumor in an animal.

US 2010/285573 describes an apparatus and a method of processing a biological and/or chemical sample in a liquid droplet. The apparatus includes a processing compartment, which is defined by a reservoir and an immobilisation member. A liquid droplet is disposed onto the predefined immobilisation area. A process is performed on the biological and/or chemical sample in said liquid droplet.

Edgington, et al. (Current Opinion in Chemical Biology 15 (2011)) describe functional imaging proteases and advances in the design and application of substrate-based and activity-based probes.

### SUMMARY

The present invention is defined by independent claim 1. The dependent claims depict other embodiments of the invention.

Provided herein are methods of detecting protease activity in a complex biological sample, in particular methods of detecting degradative and non-degradative enzymatic activity in a complex biological sample without sample processing. In some embodiments, the complex biological sample is selected from among blood, plasma, and serum.

In accordance with the invention, provided herein is a method of identifying protease activity in a complex biological sample, comprising:a) incubating the complex sample with a synthetic peptide library to generate cleavage products, wherein the synthetic peptide library comprises combinations of amino acid pairs, positively and negatively charged residues, or modified residues at different positions along the length of the synthetic peptides, and a fluorescent tag; b) separating the cleavage products from the complex sample; and d) generating a proteolytic signature for the complex sample; wherein the synthetic peptide library comprises synthetic charge-changing fluorescent peptide substrates specific for proteases, wherein the charge-changing peptide sequences selectively target proteases selected from coagulation related proteases, metalloproteases, cathepsins, bacterial proteases, kallikreins, and calpains, wherein the coagulation related protease is thrombin and the charge-changing peptide sequence has the sequence Ac-N-D-D-Nle-T-P-R-G-S-A-G-A-G-A-G-diamino-ethyl-Bodipy FL (BFL), wherein the metalloprotease is MMP1 and the charge-changing peptide sequence has the sequence Ac-N-D-G-P-Q-A-I-A-G-Q-G-A-G-diamino-ethyl-BFL, wherein the metalloprotease is MMP3 and the charge-changing peptide sequence has the sequence Ac-D-G-P-K-P-V-E-Nva-Y-N-K(ε-BFL)-NH₂, wherein the metalloprotease is MMP8 and the charge-changing peptide sequence has the sequence set forth in Ac-N-D-G-P-Q-G-Y-A-G-Q-G-A-G-diamino-ethyl-BFL, wherein the cathepsin is cathepsin-S and the charge-changing peptide sequence has the sequence N-SUC-E-G-R-W-H-T-V-G-L-R-W-E-C(Cy5)-R-CO-NH₂, wherein the cathepsin is cathepsin-D and the charge-changing peptide sequence has the sequence N-SUC-D-D-L-V-V-L-F-V-K-K-C(Cy5)-A-CO-NH₂, wherein the bacterial protease is Omp-T and the charge-changing peptide sequence has the sequence Ac-SUC-D-G-D-K-Y-R-R-A-W-G-D-T-I-diamino-ethyl-BFL, wherein the bacterial protease is SspB and the charge-changing peptide sequence has the sequence Ac-D-G-D-A-F-S-K-A-L-P-K(ε-BFL)-NH₂, wherein the kallikrein is kallikrein 2 and the charge-changing peptide sequence has the sequence Ac-N-D-G-D-T-F-R-S-A-A-G-K(ε-BFL)-NH₂, wherein the kallikrein is kallikrein 3 and the charge-changing peptide sequence has the sequence Ac-N-D-G-S-S-I-Y-Q-S-S-T-G-diamino-ethyl-BFL, or wherein the calpain is calpain 1 or calpain 2 and the charge-changing peptide sequence has the sequence N-SUC-E-P-L-F-A-A-R-K(ε-BFL)-NH₂.

In some embodiments of the method, the complex biological sample is whole blood.

In some embodiments of the method, the cleavage products are separated from the complex sample by gel electrophoresis, capillary electrophoresis, or a combination of DC and AC electrokinetic techniques.

In some embodiments of the method, the substrate signature is generated by detecting and ranking individual substrates in the peptide mixture according to migration. In some embodiments, the individual substrates are detected via the tag. In some embodiments, the tag is a fluorescent dye, a radioactive probe, or an affinity tag.

In some embodiments of the method, the proteolytic signature comprises multiple classes of protease activity. In some embodiments, the multiple classes of protease activity are selected from serine, cysteine, threonine, aspartyl, and metallopeptidase activity. In some embodiments, the multiple classes of protease activity are detected by separately incubating the complex sample with an activity inhibitor. In some embodiments, the activity inhibitor is selected from a metal chelator, a cysteine peptidase inhibitor, and an elastase-specific inhibitor. In some embodiments, the activity inhibitor is selected from EDTA, E-64, CAO74, and a chloromethyl ketone inhibitor.

### DESCRIPTION OF DRAWINGS

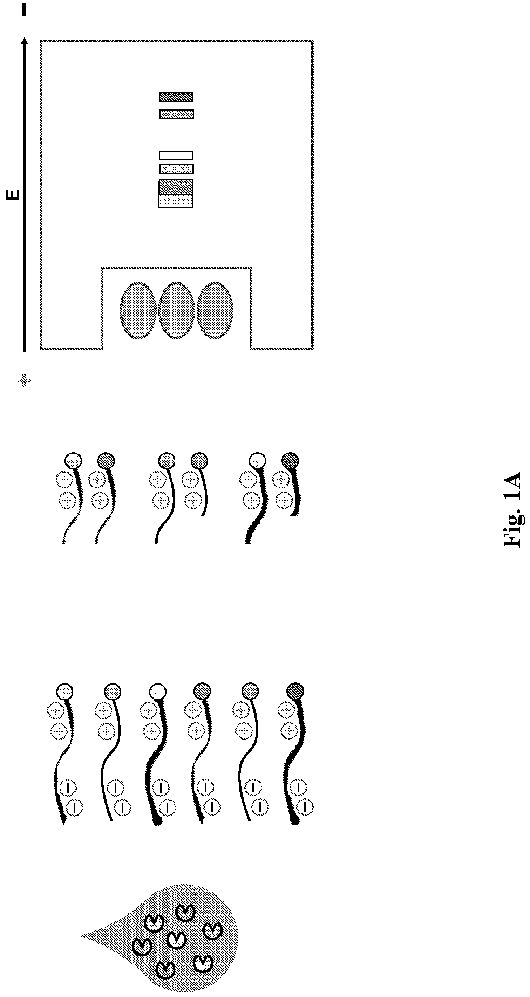

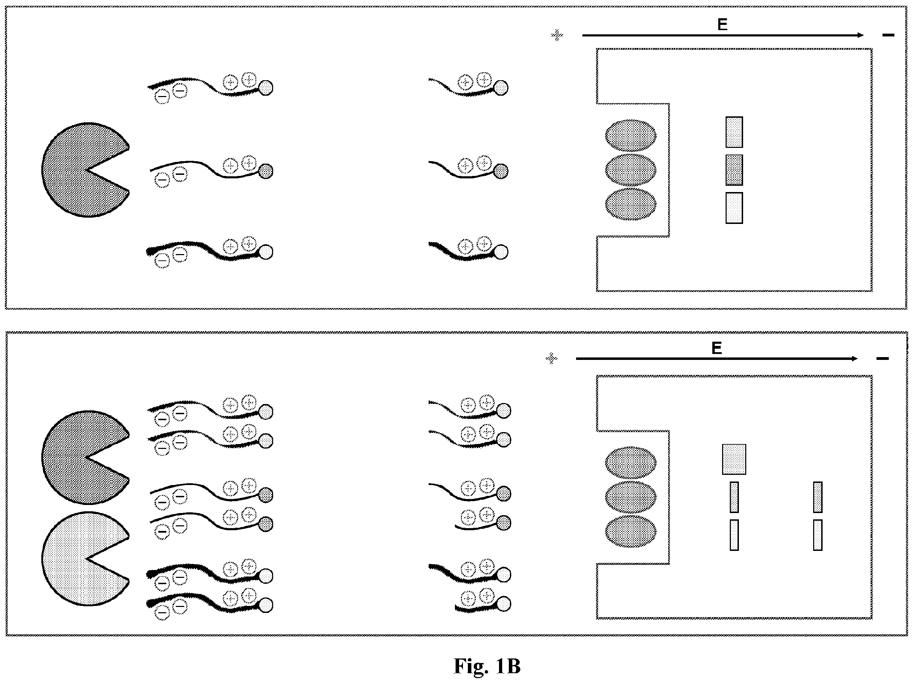

**Figures 1A-1B** show the use of substrate libraries and the resulting patterns of substrate cleavage to identify enzymes in a sample. **Figure 1A** illustrates the use of non-targeted libraries of substrates and the resulting patterns of substrate cleavage to identify multiple enzymes in samples. **Figure 1B** illustrates substrate panels and resulting patterns of substrate cleavage to differentiate enzymes with overlapping cleavage specificities such as MMP-2 (top panel) and MMP-9 (bottom panel).

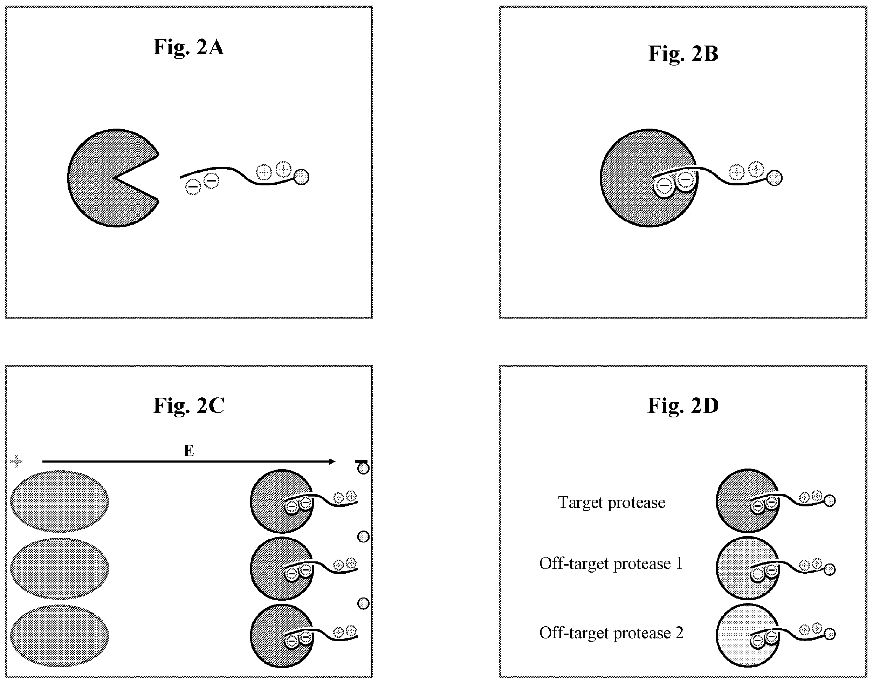

**Figures 2A-2D** show activity-based probes with charged moieties for protease purification. **Figure 2A** shows a protease inhibitor molecule with a negatively charged inhibiting region and a positively charged tail. **Figure 2B** shows that the protease binds the inhibitor molecule and masks the negatively charged inhibiting region while the tail remains exposed with positive charge, but does not cleave the molecule. **Figure 2C** shows application of an electric field to the sample to separate the negatively charged blood components from the positively charged protease/inhibitor. **Figure 2D** shows that the method is used to screen inhibitor molecules in complex samples by separating all inhibited proteases and characterizing for off-target proteases.

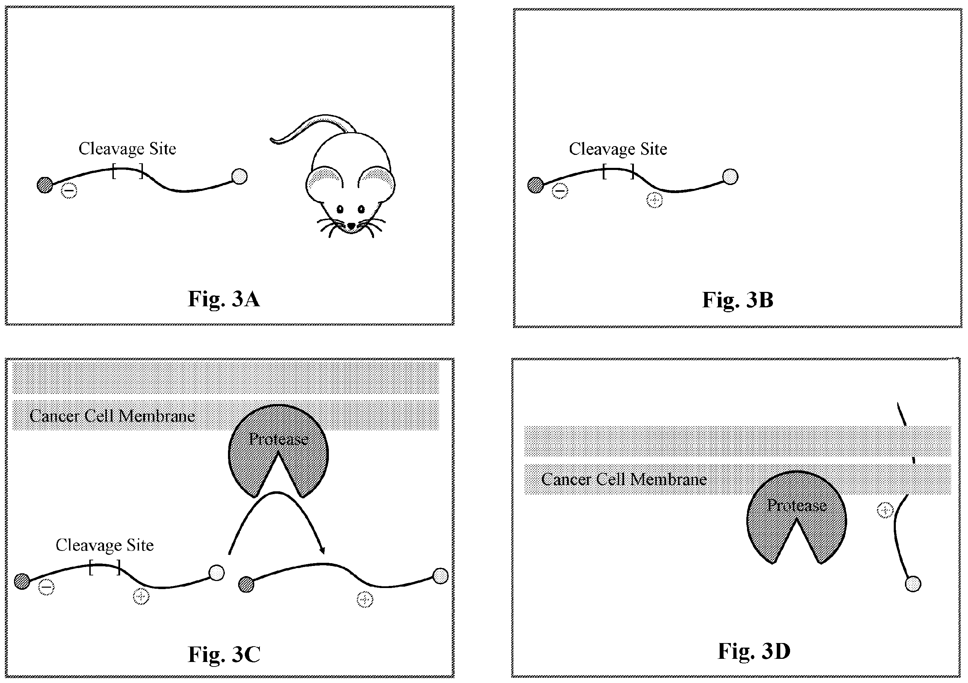

**Figures 3A-3D** show *in vivo* imaging of protease activity and localization using charge-changing substrates. **Figure 3A** shows injection of a FRET peptide into an animal that is negatively-charged to provide better transport in the bloodstream. **Figure 3B** shows that upon reaching the tumor microenvironment, the acidity of the tumor microenvironment protonates histidine, rendering the peptide neutral and able to penetrate into the tumor. **Figure 3C** shows the peptide, designed to be cleaved by proteases associated with the tumor microenvironment, is cleaved and unquenched. **Figure 3D** shows that the positively charged cleavage product is "sticky" to the cell surface. Fluorescent signal indicates tumor protease activity and tumor protease localization.

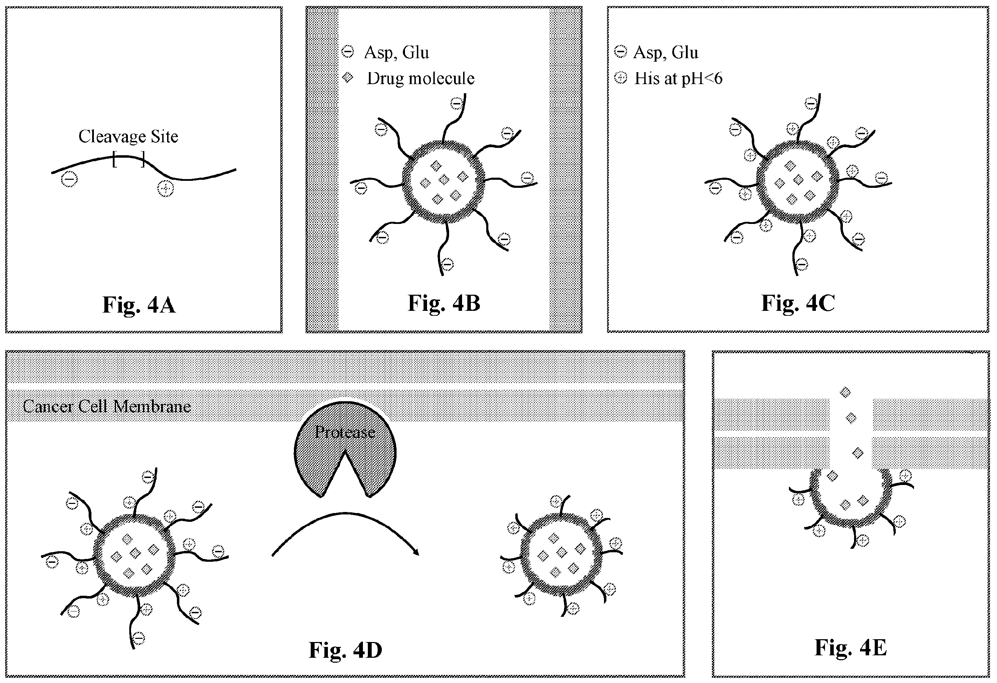

**Figures 4A-4E** show 3-step charge-changing substrates that enhance protease detection, imaging, and payload delivery in acidic microenvironments of tumors. **Figure 4A** shows a peptide designed to contain a cleavage site for a protease associated with cancer that is displayed at the cancer cell surface. The peptide is designed to change charge with tumor-associated pH change and cancer cell surface-associated proteolytic cleavage to enhance nanoparticle (NP) delivery. **Figure 4B** shows the negatively charged NP in the bloodstream for stealth in circulation. **Figure 4C** shows the NP is neutral in the acidic tumor microenvironment and remains slippery to diffuse deep into the tumor. **Figure 4D** shows a protease at the cancer cell surface cleaving the peptide at the NP surface, leaving the NP positively charged for sticky cell binding. **Figure 4E** shows a positively charged NP sticks to cancer cell membranes and is electrostatically adsorbed, triggering cellular uptake.

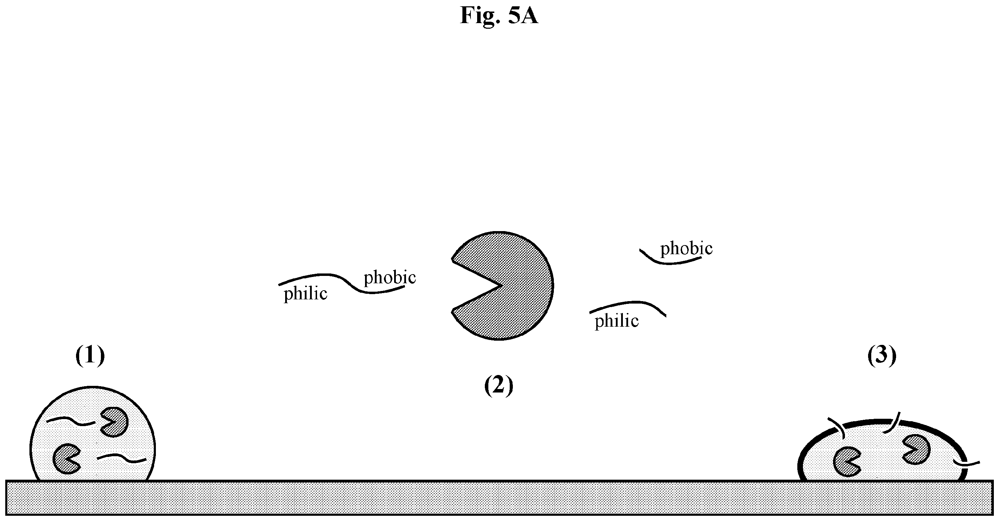

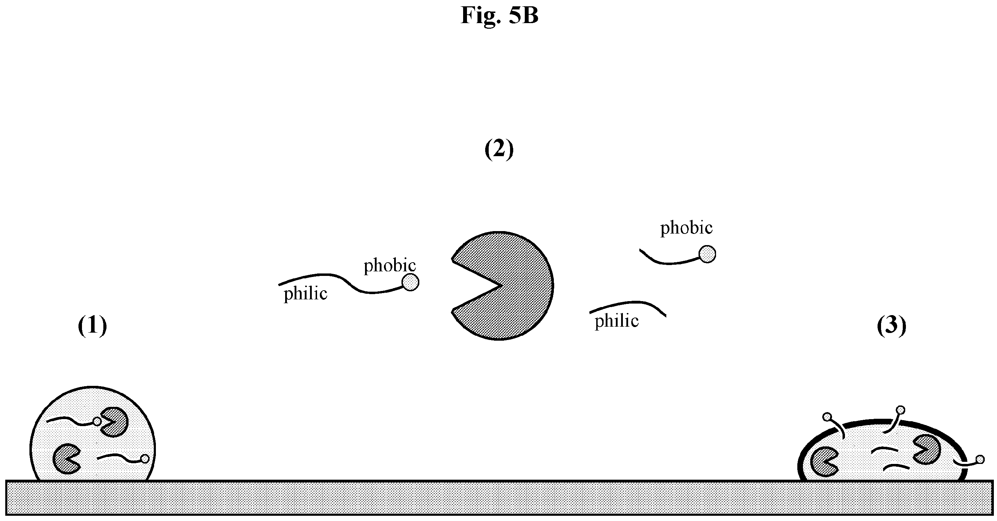

**Figures 5A-5B** illustrate detection of amphiphilic enzyme substrates. **Figure 5A** shows the contact angle of sample droplet indicates protease activity yielding hydrophobic products. (1) The sample is incubated with a substrate that balances hydrophobicity and hydrophilicity around a protease cleavage site; (2) proteases cleave substrates, producing hydrophilic and hydrophobic products; and (3) hydrophobic products migrate to the air/liquid interface and reduce the contact angle of the sample droplet, indicating proteolytic activity. **Figure 5B** shows the fluorescent hydrophobic cleavage products assemble at surface of droplet where fluorescence is not quenched by components of droplet. (1) The sample is incubated with a substrate that balances hydrophobicity and hydrophilicity around a protease cleavage site; (2) proteases cleave substrates, producing hydrophilic and hydrophobic products; and (3) hydrophobic products migrate to the air/liquid interface and fluorescence is displayed on the surface of the droplet, indicating proteolytic activity.

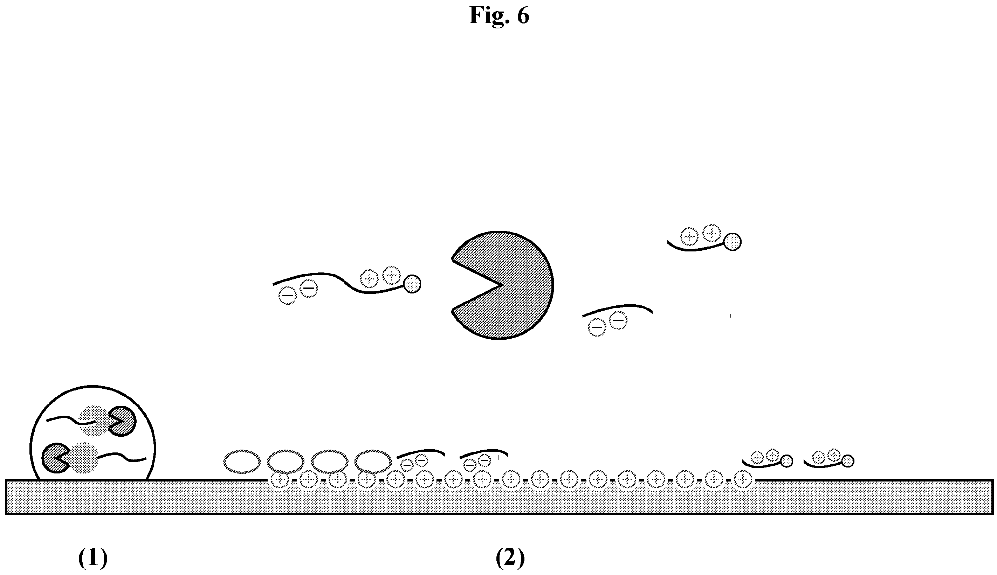

**Figure 6** is a lateral flow assay / thin layer chromatography of charge-changing substrate, showing (1) a neutral surface for sample incubation with the substrate and (2) a cationic surface that binds negative components of blood and causes positive cleavage product to associate with the liquid phase more than the solid phase.

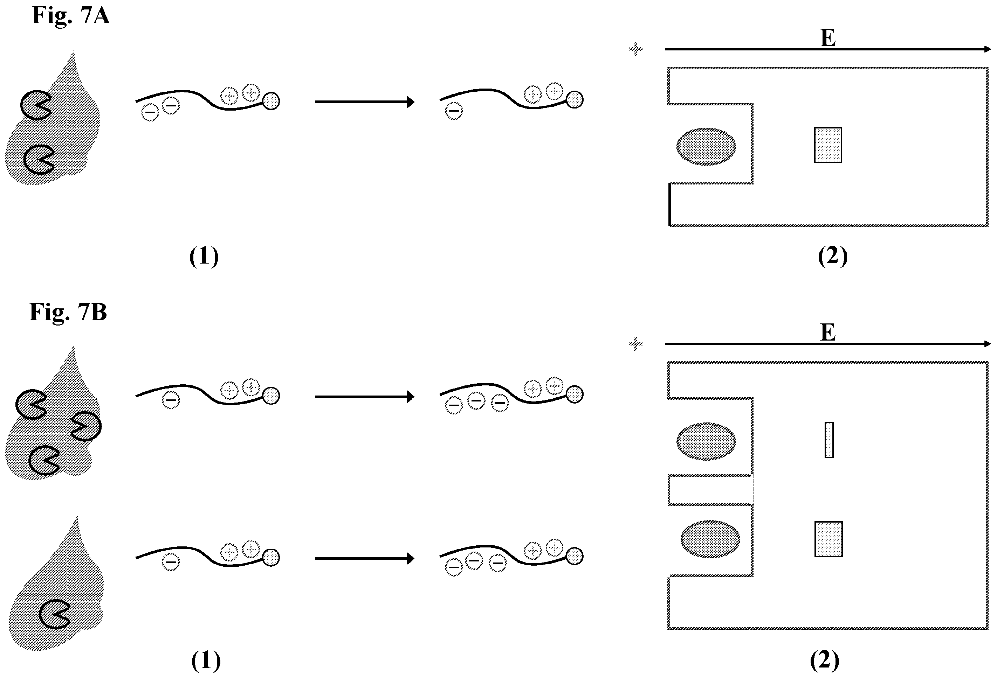

**Figures 7A-7B** shows the detection of non-degradative enzymes such as phosphorylases and kinases using charge-changing substrates and electrophoretic separation from unprocessed sample. **Figure 7A** shows (1) a phosphorylase removing negatively charged phosphate groups from a substrate, such as a phosphorylated serine group and (2) electrophoretic separation of the positively charged dephosphorylated substrate. **Figure 7B** shows (1) kinases adding a negatively charged phosphate group to a substrate and (2) comparison between samples with kinase activity of negatively charged phosphorylate substrate that has reduced fluorescence.

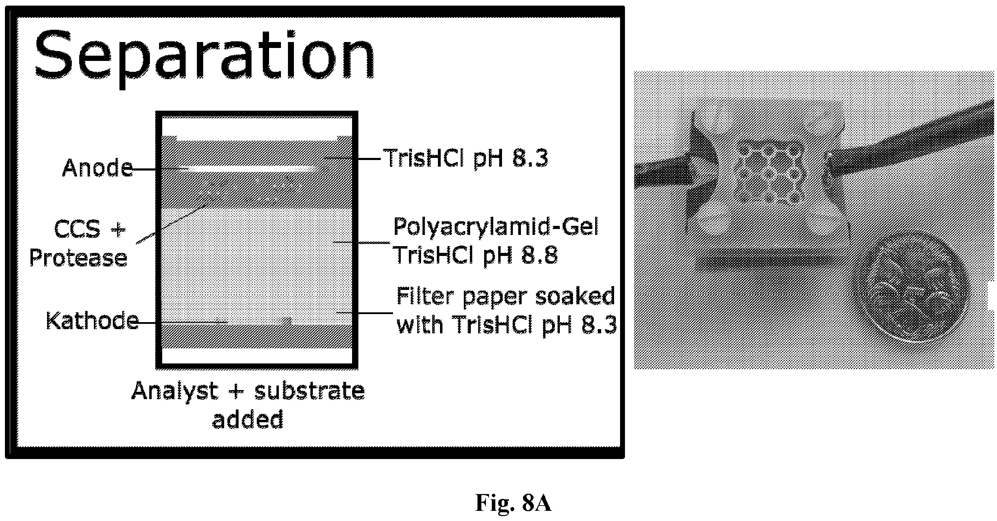

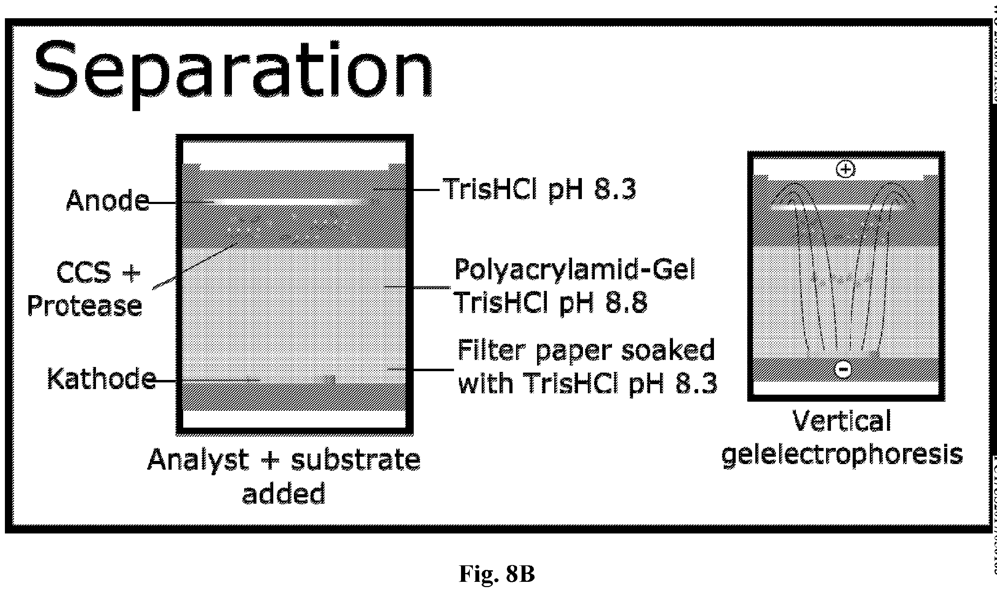

**Figures 8A-8B** show an electrophoresis focusing assay.

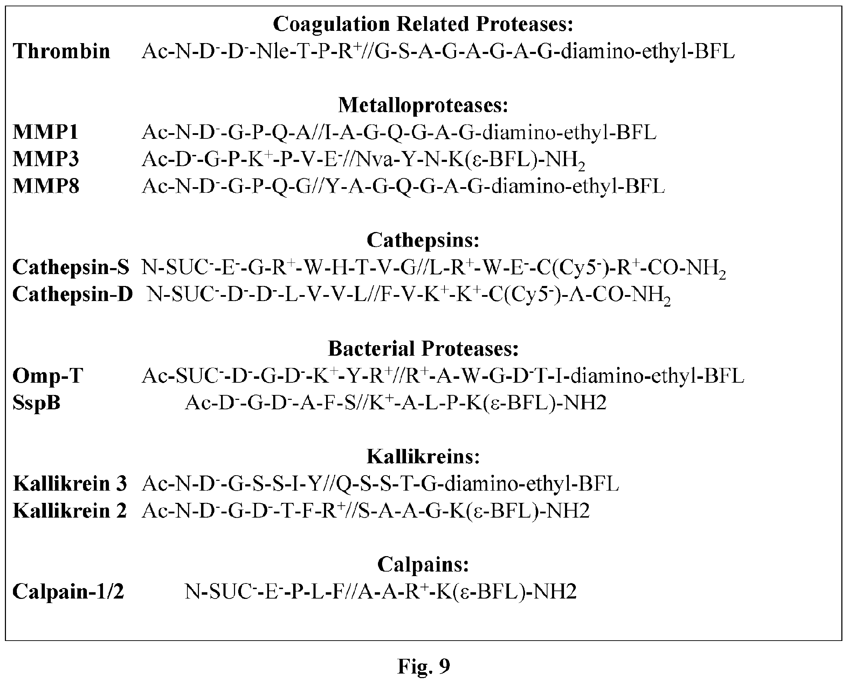

**Figure 9** shows charge-changing peptide sequences that selectively target proteases that include coagulation related proteases (thrombin), metalloproteases (MMP1, MMP3, and MMP8), cathepsins (cathepsin-S and cathepsin-D), bacterial proteases (Omp-T and SspB), kallikreins (kallikrein 2 and kallikrein 3), and calpains (calpain 1 and calpain 2).

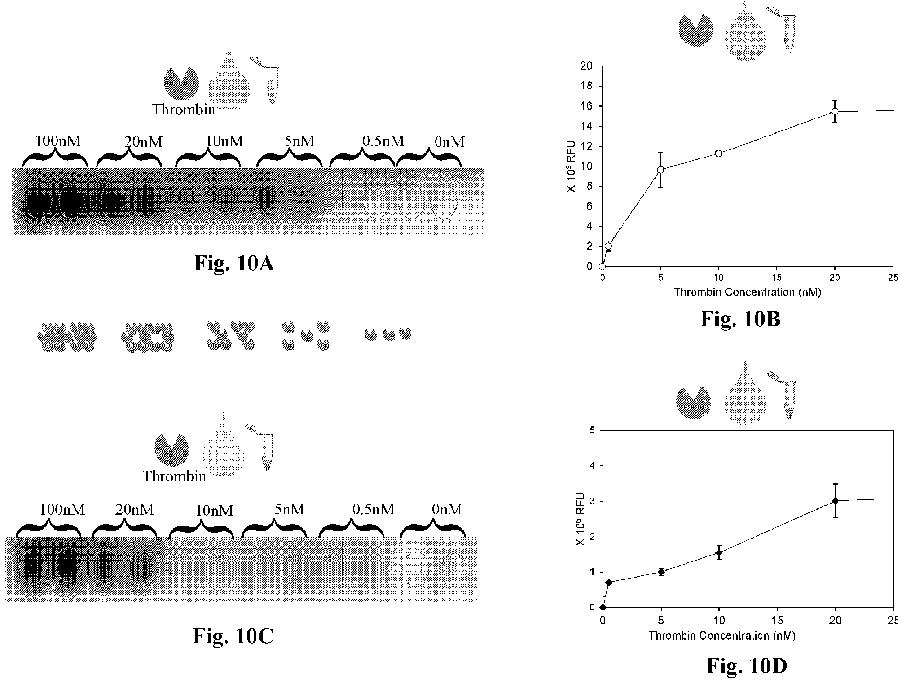

**Figures 10A-10D** show thrombin detection using a thrombin-specific charge-changing peptide substrate. **Figure 10A** shows incubation of thrombin and the peptide substrate in 1xPBS. **Figure 10B** is a graph showing thrombin concentration after incubation in 1xPBS. **Figure 10C** shows incubation of thrombin and the peptide substrate in citrated blood. **Figure 10D** is a graph showing thrombin concentration after incubation in citrated blood.

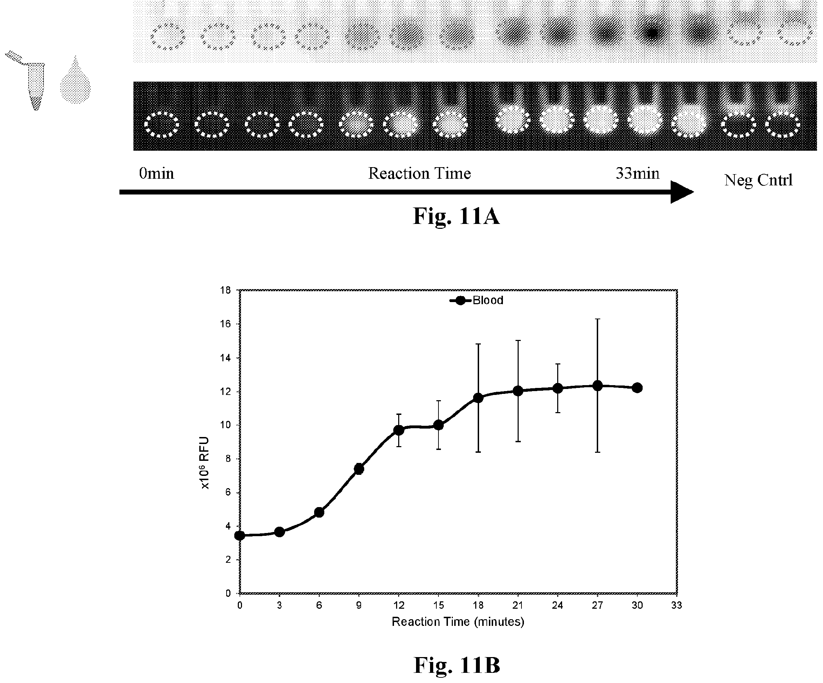

**Figures 11A-11B** show whole blood thrombin detection using a thrombin-specific charge-changing peptide substrate. **Figure 11A** illustrates fluorescence signal at various incubation time points. As compared to a negative control. **Figure 11B** is a graph showing fluorescence intensity at various incubation time points.

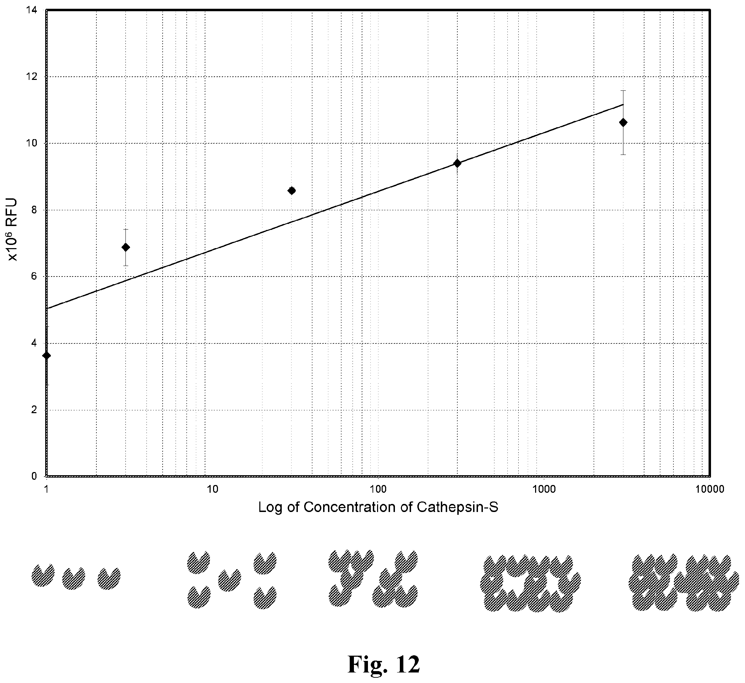

**Figure 12** shows cathepsin-S in buffer (1xPBS) detection using a cathepsin-S specific charge-changing fluorescent substrate.

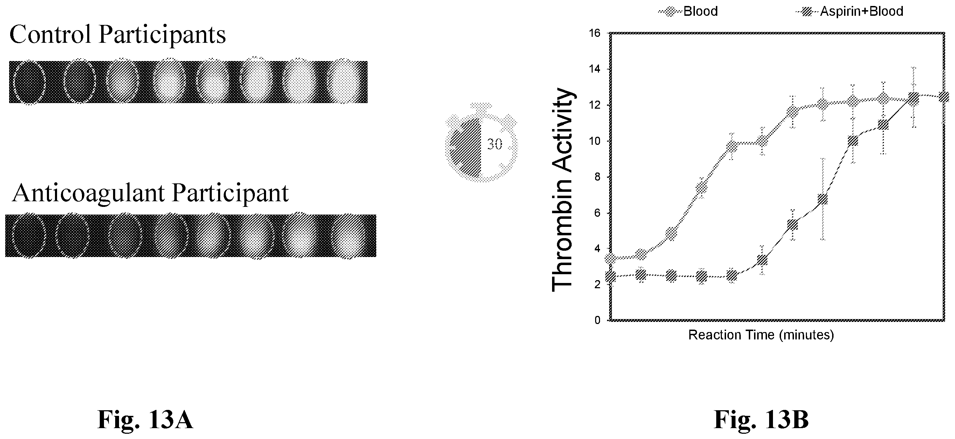

**Figures 13A-13B** illustrate thrombin activity in blood samples from subjects taking an anticoagulant (aspirin) vs. control. **Figure 13A** shows fluorescence after time in control subjects (top panel) vs. anticoagulant subjects (bottom panel). **Figure 13B** is a graph showing thrombin activity over time.

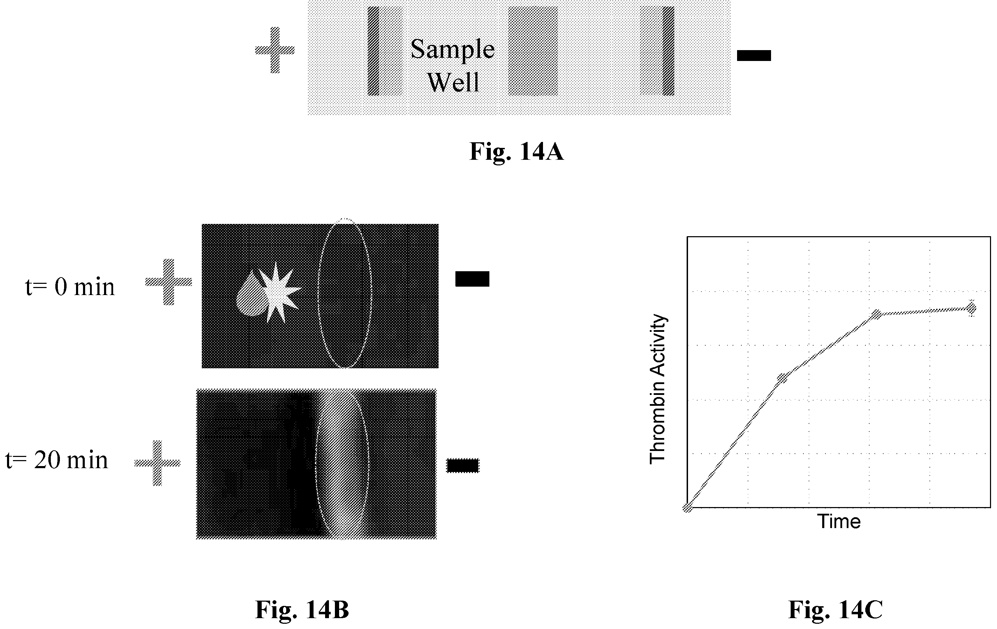

**Figures 14A-14C** illustrate a prototype for coagulation monitoring in real time. A sample is placed in a sample well with a peptide substrate (e.g., a charge-changing peptide substrate) **(****Figure 14A****),** fluorescence is measured at t₀ **(****Figure 14B****,** top panel) and again at a later time points **(****Figure 14B****,** bottom panel). **Figure 14C** shows that thrombin activity can be monitored in real time.

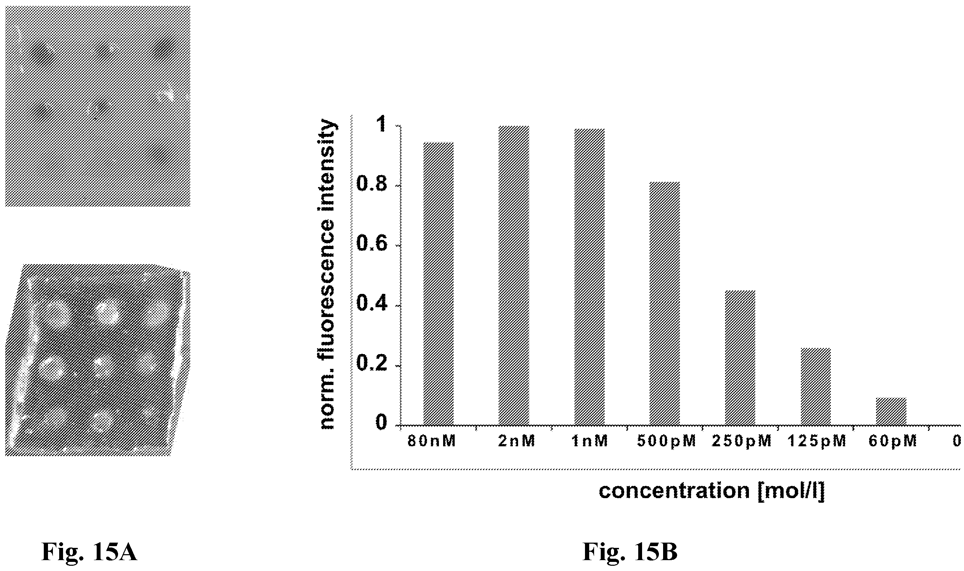

**Figures 15A-15B** show a miniaturized array prototype. **Figure 15A** shows a miniaturized array prototype. **Figure 15B** shows the results of an electrophoresis focusing assay measuring protease (trypsin) activity as fluorescence intensity vs. concentration.

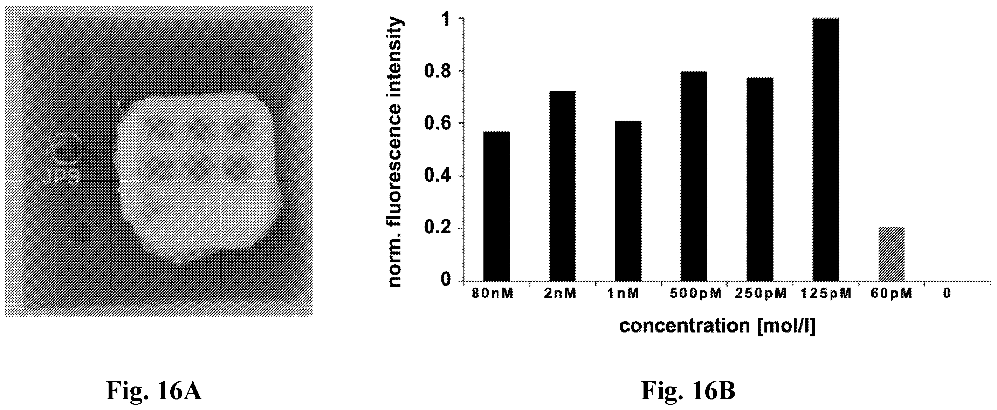

**Figures 16A-16B** show a miniaturized array prototype. **Figure 16A** shows a miniaturized array prototype. **Figure 16B** shows the results of an electrophoresis focusing assay measuring protease (trypsin) activity after one week as fluorescence intensity vs. concentration.

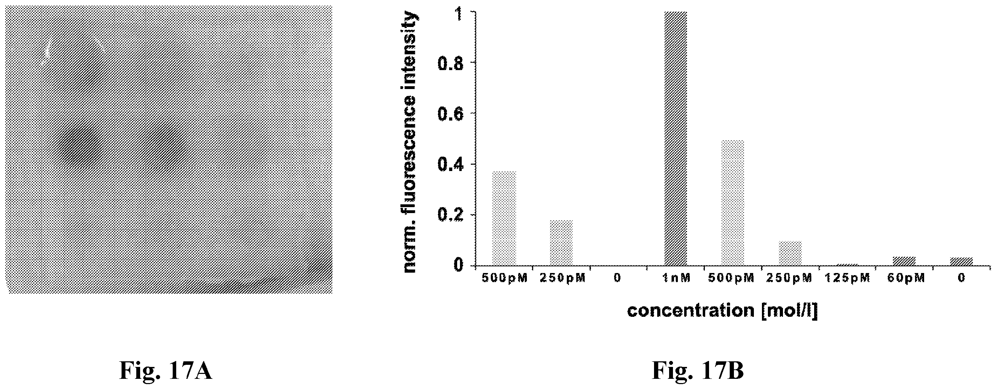

**Figures 17A-17B** show a miniaturized array prototype. **Figure** 17A shows a miniaturized array prototype. **Figure 17B** shows the results of an electrophoresis focusing assay measuring protease (trypsin) activity in a blood sample as fluorescence intensity vs. concentration.

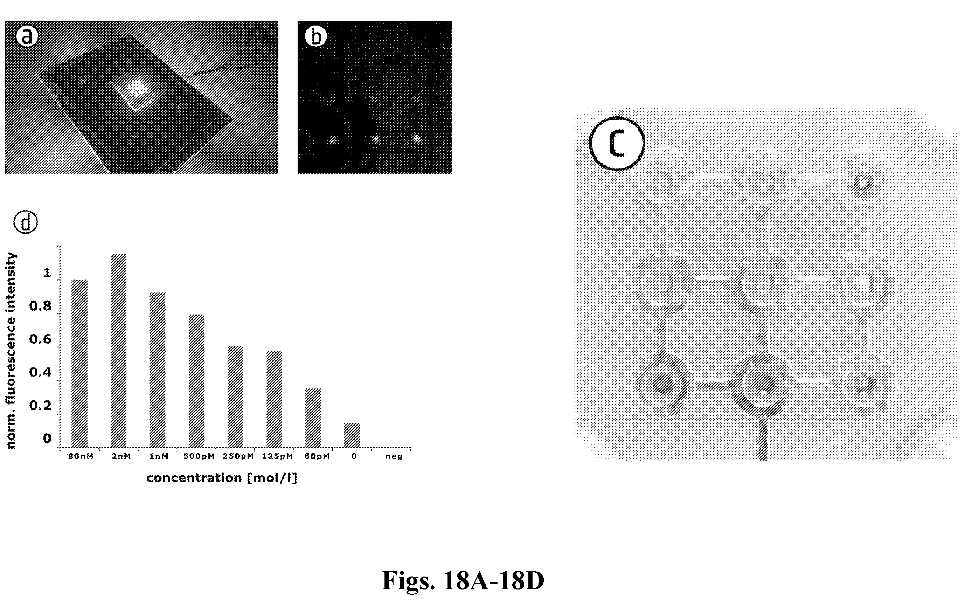

**Figures 18A-D** show a miniaturized array prototype and results of an electrophoresis focusing assay.

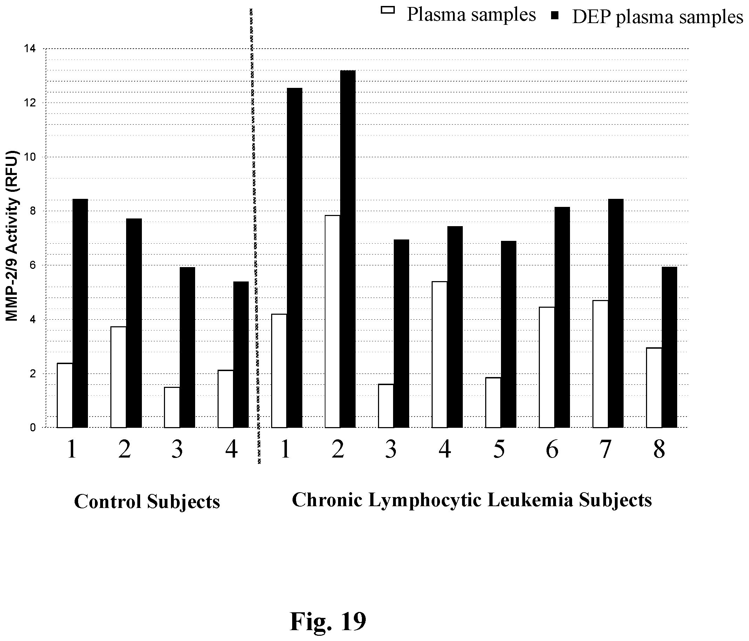

**Figure 19** shows detection of MMP2 and MMP activity in plasma samples from chronic lymphocytic leukemia subjects vs. control in plasma samples (white bars) and plasma samples subjected to dielectrophoretic (DEP) separation (black bars).

### DETAILED DESCRIPTION

Provided herein are methods of detecting degradative and non-degradative enzymatic activity in complex biological samples without sample processing. In some embodiments, targeted or non-targeted libraries of property-changing (e.g., charge, hydrophobicity) enzyme substrates are separated from the biological sample based on their change in properties upon enzymatic action, for example, cleavage or moiety addition/subtraction. In some embodiments, separation of property-changing (e.g., charge, hydrophobicity) enzyme substrates is by electro kinetic methods, chromatographic techniques, and *in situ* assembly at hydrophobic-hydrophilic, e.g. air-liquid, interfaces. In some embodiments, detection of cleavage products of charge-changing and hydrophobicity-changing enzyme substrates is done using fluorescent/colorimetric dyes, impedance/capacitance detectors, contact angle, and turbidity/light absorption measurement to indicate enzymatic activity in complex samples. In some embodiments, the complex biological sample is selected from among blood, plasma, and serum. In some embodiments, formats including non-targeted library platforms, 96-well plates, lateral flow assays, cassettes, skin patches, and microarrays adapted for separation based on charge/hydrophobicity changes are used.

As an example not forming part of the claimed invention, also provided herein are methods of enabling the purification of enzymes, e.g. proteases, from complex samples without sample processing. In some embodiments, the complex biological sample is selected from among blood, plasma, and serum. In some embodiments, charge-balanced probes that inhibit/bind the cleavage/allosteric site of the enzyme through their negatively charged section are used. In some embodiments, the enzyme shields this bound negatively charged section of the probe while the positively charged section of the probe is not shielded and used to separate out the enzyme from the complex sample under the action of an electric field. In some embodiments, the complex biological sample is selected from among blood, plasma, and serum.

In some embodiments, an assay technique is used to detect and measure proteases directly in a few microliters of whole blood. In some embodiments, the protease is selected from among indigenous and nonindigenous proteases including, but not limited to trypsin, chymotrypsin, MMPs, and combinations thereof. In some embodiments, the protease is thrombin. Many indigenous proteases like thrombin are important for blood clotting activities and related cardiac diseases (DVT, atherosclerosis, etc.). The appearance of certain nonindigenous proteases like trypsin, chymotrypsin, and MMPs are frequently biomarkers for shock, cancer and other diseases. Thus, the ability to measure protease activity in whole blood will allow researchers to further elucidate the relationship between circulating protease levels and many important diseases.

Provided herein are synthetic charge-changing fluorescent peptide substrates which are specific for proteases. In some embodiments, the protease is selected from trypsin, chymotrypsin, MMP2, MMP9, elastase, and thrombin. In some embodiments, the peptide substrates allow direct detection of protease activity in a complex biological sample and eliminate the need for sample preparation. In some embodiments, the complex biological sample is selected from among blood, plasma, and serum. In some embodiments, the substrates are added to a few microliters of a complex sample for reaction, and the cleaved products are then rapidly separated in a simple electrophoretic microgel format. In some embodiments, the complex samples is blood.

In some embodiments, the target of the invention are catalytic enzymes, including, but not limited to proteases, lipases, nucleases, and non-catalytic enzymes. In some embodiments, the targets are proteases.

In some embodiments of the assays and methods provided herein, the sample to be analyzed is a biological sample. In some embodiments, the input material is a biological sample such as blood, saliva, cerebro-spinal fluid, or urine. In some embodiments, the biological sample is blood. In some embodiments, the sample does not require special processing before being placed into the process.

In some embodiments, the assays and methods provided herein can be performed in laboratory ware. In some embodiments, the laboratory ware includes a 96-well pate or flask, a reaction container such as a cassette, or a lateral flow device similar to a pregnancy test kit, etc. In some embodiments, a patch-type device for placing on a person that allows for continuous monitoring is also considered.

In some embodiments, the reacting elements are chemicals. In some embodiments, the reacting elements include, but are not limited to, synthesized sequences of peptides, DNA, and RNA. In some embodiments, the chemicals carry 'tags' to facilitate the process. In some embodiments, the chemicals are engineered to bear hydrophilic and hydrophobic regions, or positively and negatively charged regions, although other differentiating features could be chosen. In some embodiments there is a single chemical used in the process. In some embodiments there is a plurality of chemicals, where such plurality can be chosen for a unique combination of reaction outputs. These plurality of chemicals would comprise a 'library' of available molecules for use.

In some embodiments the tags are fluorescent. In some embodiments, the tags are luminescent or radioactive. In some embodiments, the tag includes a sequence that is dominated by entities bearing specific physio-chemical properties, such as charge or hydro-phobicity/philicity.

In some embodiments of the assays and methods provided herein, the reaction that occurs is a chemical reaction. In some embodiments, the chemical reaction is proteolytic activity by the enzymes present in the biological sample digesting/degrading the tag-bearing-chemicals. In some embodiments, the chemical reaction is binding or another reaction. The tag-bearing chemicals function as substrates for the enzymes, or as protease-specific peptides. In some embodiments, the reaction cleaves the peptides, releasing a hydrophobic, or highly-charged, or otherwise 'polarized' entity. Such an entity possesses properties that can be separated and measured, for useful purposes. Alternatively, in some embodiments, the tag-bearing chemicals function to bind a region or pocket of the enzyme in the sample, where such binding exposes only one portion of the chemical, where such exposure allows a method of separation and measurement.

In some embodiments, following the reaction, a processing step can occur. In some embodiments, the processing step is an electrophoretic process, chromatography, lateral flow, or hydrophobic moieties moving to the surface of the liquid, etc. This can be a single step, or several steps. In some embodiments, this processing can also include purification or extraction of enzymes of interest.

In some embodiments, following the processing, measurement can be performed. In some embodiments, the measurement is electrical, conductivity, impedance, or colorimetric. In some embodiments, the measurement is interpreted by an operator, or by software, that enables a useful result, such as a diagnostic output, to be rendered.

### Protease activity profiling with libraries of charge-changing peptides

Functional characterization of peptidases in a biological system has traditionally involved identifying a candidate protein, determining the substrate specificity and generating a specific probe or inhibitor. Although the candidate approach has a proven track record, there is a need to characterize proteolysis using an unbiased global approach. Recently two DNA microarray technologies, the Hu/Mu ProtIn chip and the CLIP-CHIP^{®}, were developed to study proteases. However, transcript levels only indicate which proteases can potentially be found in a sample because mRNA levels do not always correlate with protein abundance and are devoid of information regarding protease activity. Proteomics offers a means to evaluate the presence of proteins and their changes in abundance within complex mixtures. However, it also fails to detect the functional state of a protein. For proteases there is a difference between abundance and activity, and a technique to track protease activity in complex samples is needed.

To study this, the concept of "reverse degradomics" was introduced, which seeks to measure the total impact of a complex biological sample on a particular substrate. Fluorescently labeled substrates are incubated with biological samples, and at different time points the degree of degradation is assessed in a high-throughput manner. This enables one to predict and compare the total impact of complex samples on, for example, key disease-related substrates, in a setting close to the *in vivo* substrate environment. However, these reverse techniques, including zymography and mass spectrometry, rely on time consuming protocols, highly specialized equipment, and manipulation of samples by processing.

### Identifying the proteolytic signature of complex samples

According to the invention, provided herein are methods of identifying the proteolytic signature of complex samples. The complex biological sample is selected from among blood, plasma, and serum. In some embodiments, provided herein is a method of generating comprehensive substrate signatures for enzyme classes in complex samples. In some embodiments, the substrate library and the resulting pattern of substrate cleavage is used to identify enzymes in a sample. For example, a synthetic peptide library containing combinations of amino acid pairs, combinations of positively and negatively charged residues or modified residues at different positions along the length of the synthetic peptides, and a tag such as a fluorescent dye can be prepared. In some embodiments, substrate signatures can be generated after an incubation of complex samples with the substrate library and separation of the cleavage products of the incubated substrate library from the complex sample by gel electrophoresis, capillary electrophoresis, or a combination of DC and AC electrokinetic techniques (Figures 1A-1B). In some embodiments, individual substrates in the peptide mixture are ranked according to migration and detected via fluorescence or other tag. In some embodiments, enzymes with overlapping cleavage specificities can be differentiated. For example, in some embodiments, MMP-2 and MMP-9 can be differentiated.

In some embodiments, to dissect the contribution of multiple classes of protease activity such as serine, cysteine, and metallopeptidases, samples are tested after being incubated separately with various activity inhibitors. In some embodiments, the activity inhibitors are the metal chelator EDTA, the cysteine peptidase inhibitors E-64 and CAO74, or the elastase-specific chloromethyl ketone inhibitor. In some embodiments, cleavage-site specificity data from inhibited samples is combined to generate a substrate signature for each peptidase class. In some embodiments, secondary synthetic peptide libraries are generated to further define the cleavage preferences of peptidases in complex samples. In some embodiments, the complex biological sample is selected from among blood, plasma, and serum.

### Protease purification with charge-changing activity-based probes

Identification and quantification of active proteases found in biological samples can be achieved by combining the use of activity-based probes (ABPs) and mass spectrometry analysis. ABPs are molecules derived from mechanism-based inhibitors. ABPs have been developed for several enzyme classes, but have proven particularly useful for the study of proteases. ABPs targeting serine proteases, cysteine proteases, threonine proteases, aspartyl proteases, and metalloproteases have been developed. ABPs irreversibly bind to active proteases but not to inactive zymogens or an inhibited enzyme. ABPs are composed of a chemically reactive group, often termed a "warhead," attached to a tag moiety via a spacer molecule. Selectivity of the probe can be increased by the addition of a specificity-enhancing module. Upon binding of the ABP to the protease active site, the chemically reactive group reacts irreversibly with the active site nucleophilic residue. The tag of ABPs allows for either visualization (fluorophore or radioactive probe) or isolation (affinity tag) of the ABP·protease complex.

However, ABP approaches rely on time consuming protocols, highly specialized equipment, and manipulation of samples by processing. There is a need for techniques that enable unamplified study of protease activity, as is accomplished with ABP as opposed to cleavable substrate methods, that also function in complex samples and do not require specialized equipment such as mass spectrometry. In some embodiments, the complex biological sample is selected from among blood, plasma, and serum.

### In vitro purification by activity based probes

In some embodiments, provided herein are activity-based probes with charged moieties for protease purification. In some embodiments, provided is an ABP design composed of a chemically reactive group attached to a positively charged moiety via a spacer molecule (Figures 2A-2D). In some embodiments, the protease inhibitor molecule has a negatively charged inhibiting region and a positively charged tail. In some embodiments, the ABP is incubated with a sample *in vitro.* In some embodiments, the ABP binds to the protease active site and the chemically reactive group reacts irreversibly with the active site nucleophilic residue. In some embodiments, the protease binds the inhibitor molecule but does not cleave the molecule. In some embodiments, the protease binds the inhibitor molecule and masks the negatively charged inhibiting region. In some embodiments, the positively charged moiety of the ABP (e.g., tail) remains exposed outside the active site of the protease.

In some embodiments, electrophoretic separation *in vitro* is used to separate the proteases that have bound ABP since the positively charged moiety will separate from the majority of components of the complex biological sample that are negatively charged. In some embodiments, the complex biological sample is selected from among blood, plasma, and serum. In some embodiments, an electric field is applied to the sample. In some embodiments, the negatively charged blood components separate from the positively charged protease/inhibitor.

In some embodiments, visualization of protease activity at the site of a tumor is possible. In some embodiments, the tumor is biopsied. In some embodiments, after biopsy the ABP-bound proteases are separated from the complex sample by electrophoretic separation. In some embodiments, ABP-bound proteases that have been separated from complex samples are available for further downstream applications and characterization of post-translational modifications, mutations, etc. In some embodiments, the complex biological sample is selected from among blood, plasma, and serum.

### Protease detection and imaging of acidic microenvironments in vivo

The introduction of whole-body imaging techniques for small animals enabled *in vivo* zymography (IVZ). This technique relies on the use of protease-activated fluorogenic probes. Specific probes have been developed containing different fluorophores, which allow simultaneous detection of various enzymes. IVZ has allowed the mapping of MMP activity patterns in an intact organism. However, IVZ probes are not designed to preferentially localize in certain microenvironments of the body, which could afford more accurate imaging of hard to access microenvironments of interest, such as the acidic microenvironments of tumors.

### Protease detection by peptides

As an example not forming part of the claimed invention,, provided are methods of imaging protease activity and localization using charge-changing substrates. In some embodiments, the protease detection technique enables *in vivo* imaging of localized protease activity in acidic microenvironments based on charge-changing fluorescent probes (Figures 3A-3D). In some embodiments, a negatively charged fluorescently labeled peptide is injected into an organism. In some embodiments, the peptide is a FRET peptide. In some embodiments, the negative charge of the peptide affords stealth in the bloodstream of the organism. In some embodiments, upon encountering the acidic microenvironment of a tumor, the histidine residues of the peptide become positively charged and the peptide is overall neutral. In some embodiments, the peptide is rendered neutral and able to penetrate the tumor. In some embodiments, the neutral charge allows the peptide to penetrate the tumor. In some embodiments, the peptide is cleaved by the protease for which its sequence was designed. In some embodiments, the peptide is designed to be cleaved by proteases associated with the tumor microenvironment. In some embodiments, the peptide is cleaved and unquenched. In some embodiments, the cleavage product is positively charged and fluorescently or near infrared labeled which causes it to stick to the cell surface and provide imaging of protease activity and localization. In some embodiments, a fluorescent signal indicates tumor protease activity and tumor protease localization.

Surface charge of nanocarriers influences their penetration into tumor tissues. The extracellular matrix presents as an effective electrostatic bandpass, suppressing the diffusive motion of both positively and negatively charge objects, which allows uncharged particles to easily diffuse through while effectively trapping charged particles. The optimal particles for delivery to tumors should be neutral after exiting the blood vessels (see, e.g., Sun Q, Radosz M, Shen Y, Chapter 3: Rational Design of Translational Nanocarriers. Functional Polymers for Nanomedicine, edited by Younqing Shen).

To deliver a sufficient drug concentration to the tumor center region lacking vascular perfusion, where the most aggressive and resistant cells reside, the nanocarrier should not release the carried drug after extravasation but should further diffuse deep into the tumor. This requires the nanocarrier to remain slippery and have as small a size as possible. Thus, it is better for the nanocarrier to be neutral and not to present any binding groups (including targeting groups) until reaching the center of the tumor.

After reaching the targeted region the nanocarrier should efficiently enter the cells for drug release. Now the same properties that impart stealth to the nanocarrier prevent it from cellular uptake by tumor cells. Nanocarriers that are negatively charged will be repelled from the cell membrane due to the electrostatic repulsion. Thus, once in the tumor, the carrier must become cell-binding or sticky to targeting tumor cells for fast cellular uptake. The challenge is how to reconcile these two opposite requirements, stealth for circulation and diffusion versus sticky for targeting. For instance, it is well known that positively charged carrier reliably stick to cell membranes due to electrostatic adsorption triggering fast cellular uptake, but positively charged carriers are not suitable for *in vivo* applications because they are systemically toxic and have short circulation time (see, e.g., Sun Q, Radosz M, Shen Y, Chapter 3: Rational Design of Translational Nanocarriers. Functional Polymers for Nanomedicine, edited by Younqing Shen).

Positive charges can promote carrier adsorption on the negatively charged membrane and hence trigger adsorption-mediated endocytosis. Thus, an alternative is to use tumor extracellular acidity to impart positive charges to the carrier by a "charge-reversal" technique.

### In vivo protease detection by peptide decorated nanoparticles

In some embodiments, provided is a protease detection technique that enables *in vivo* imaging of localized protease activity in acidic microenvironments. In some embodiments, the method uses nanoparticles decorated with charge-changing probes (Figures 4A-4E). In some embodiments, the peptide is designed to contain a cleavage site for a protease associated with cancer. In some embodiments, the protease associated with cancer is displayed at the cancer cell surface. In some embodiments, the peptide is designed to change charge with a tumor-associated pH change. In some embodiments, the peptide is designed to change charge with cancer cell surface-associated proteolytic cleavage. In some embodiments, the charge change and cleavage enhance nanoparticle delivery.

As an example not forming part of the claimed invention, a nanoparticle decorated with negatively charged peptides is injected into an organism. In some embodiments, the negative charge of the peptide affords stealth in the bloodstream of the organism. In some embodiments, upon encountering the acidic microenvironment of a tumor, the histidine residues of the peptide become positively charged and the peptide is overall neutral, which allows the decorated nanoparticle to penetrate the tumor. In some embodiments, the peptide on the surface of the nanoparticle is cleaved by the protease for which its sequence was designed. In some embodiments, the cleavage product is positively charged which causes the decorated nanoparticle to stick to the cell surface. In some embodiments, the positively charged nanoparticle is electrostatically adsorbed and triggers cellular uptake. In some embodiments, the nanoparticle releases its payload of imaging agents and/or drug molecules into the tumor cell.

In some embodiments of the methods provided herein, the charge-changing substrates enhance protease detection. In some embodiments of the methods provided herein, the charge-changing substrates enhance protease imaging. In some embodiments of the methods provided herein, the charge-changing substrates enhance payload delivery in acidic microenvironments of tumors.

In some embodiments of the methods provided herein, the charge-changing peptide sequences selectively target proteases selected from among coagulation related proteases, serine proteases, metalloproteases, cathepsins, bacterial proteases, kallikreins, and calpains (Figure 9). In some embodiments, the coagulation related protease is thrombin and the charge-changing peptide sequence has the sequence Ac-N-D⁻-D⁻-Nle-T-P-R⁺//G-S-A-G-A-G-A-G-diamino-ethyl-BFL. In some embodiments, the serine protease is trypsin. In some embodiments, the metalloprotease is selected from among MMP1, MMP2, MMP3, MMP8, and MMP9. In some embodiments, the metalloprotease is MMP1 and the charge-changing peptide sequence has the sequence Ac-N-D⁻-G-P-Q-A//I-A-G-Q-G-A-G-diamino-ethyl-BFL. In some embodiments, the metalloprotease is MMP2. In some embodiments, the metalloprotease is MMP3 and the charge-changing peptide sequence has the sequence Ac-D⁻-G-P-K⁺-P-V-E⁻//Nva-Y-N-K(ε-BFL)-NH₂. In some embodiments, the metalloprotease is MMP8 and the charge-changing peptide sequence has the sequence Ac-N-D⁻-G-P-Q-G//Y-A-G-Q-G-A-G-diamino-ethyl-BFL. In some embodiments, the metalloprotease is MMP9. In some embodiments, the cathepsin is selected from among cathepsin-S and cathepsin-D. In some embodiments, the cathepsin is cathepsin-S and the charge-changing peptide sequence has the sequence N-SUC⁻-E⁻-G-R⁺-W-H-T-V-G//L-R⁺-W-E⁻-C(Cy5⁻)-R⁺-CO-NH₂. In some embodiments, the cathepsin is cathepsin-D and the charge-changing peptide sequence has the sequence set forth in N-SUC⁻-D⁻-D⁻-L-V-V-L//F-V-K⁺-K⁺-C(Cy5⁻)-A-CO-NH₂. In some embodiments, the bacterial protease is selected from among Omp-T and SspB. In some embodiments, the bacterial protease is Omp-T and the charge-changing peptide sequence has the sequence Ac-SUC⁻-D⁻-G-D⁻-K⁺-Y-R⁺//R⁺-A-W-G-D⁻T-I-diamino-ethyl-BFL. In some embodiments, the bacterial protease is SspB and the charge-changing peptide sequence has the sequence Ac-D⁻-G-D⁻-A-F-S//K⁺-A-L-P-K(ε-BFL)-NH2. In some embodiments, the kallikrein is selected from among kallikrein 2 and kallikrein 3. In some embodiments, the kallikrein is kallikrein 2 and the charge-changing peptide sequence has the sequence Ac-N-D⁻-G-D⁻-T-F-R⁺//S-A-A-G-K(ε-BFL)-NH2. In some embodiments, the kallikrein is kallikrein 3 and the charge-changing peptide sequence has the sequence Ac-N-D⁻-G-S-S-I-Y//Q-S-S-T-G-diamino-ethyl-BFL. In some embodiments, the calpain is selected from among calpain 1 and calpain 2. In some embodiments, the calpain is calpain 1 or calpain 2 and the charge-changing peptide sequence has the sequence N-SUC⁻-E⁻-P-L-F//A-A-R⁺-K(ε-BFL)-NH2.

### Detection of amphiphilic enzyme substrates

### Contact angle measurement

As an example not forming part of the claimed invention, provided herein are methods of detecting amphiphilic enzyme substrates. In some embodiments, the method includes measuring the contact angle of a sample droplet. In some embodiments, provided is a technique to detect protease activity in droplets of complex samples. In some embodiments, the complex biological sample is selected from among blood, plasma, and serum. In some embodiments, protease activity is detected by contact angle measurement (Figure 5A). In some embodiments, the contact angle of a sample droplet indicates protease activity. In some embodiments, the protease activity yields hydrophobic products. In some embodiments, amphiphilic substrates are designed to contain a hydrophobic and hydrophilic section around a cleavage site for a protease of interest. In some embodiments, the substrates are incubated with a droplet of the complex sample. In some embodiments, the complex biological sample is selected from among blood, plasma, and serum. In some embodiments, proteolytic cleavage occurs. In some embodiments, proteolytic cleavage produces hydrophilic and hydrophobic products. In some embodiments, hydrophilic and hydrophobic products are released from the amphiphilic substrate as proteolytic cleavage occurs. In some embodiments, the hydrophobic cleavage products preferentially assemble at the droplet-air interface at the surface of the sample droplet. In some embodiments, the contact angle of the droplet decreases as more hydrophobic cleavage products migrate to the surface of the droplet. In some embodiments, the contact angle is measured as an indicator of protease activity.

### Detection on droplet surface

As an example not forming part of the claimed invention, provided herein are methods of detecting amphiphilic enzyme substrates. In some embodiments, the method includes measuring fluorescence of a sample droplet. In some embodiments, provided is a technique to detect protease activity in droplets of complex samples. In some embodiments, the complex biological sample is selected from among blood, plasma, and serum. In some embodiments, protease activity is detected by fluorescence detection at the surface of the droplet (Figure 5B). In some embodiments, fluorescently labeled amphiphilic substrates are designed to contain a hydrophobic and hydrophilic section around a cleavage site for a protease of interest. In some embodiments, the substrates are incubated with a droplet of a complex sample. In some embodiments, the complex biological sample is selected from among blood, plasma, and serum. In some embodiments, proteolytic cleavage occurs. In some embodiments, proteolytic cleavage produces hydrophilic and hydrophobic products. In some embodiments, hydrophilic and hydrophobic products are released from the amphiphilic substrate as proteolytic cleavage occurs. In some embodiments, the fluorescently labeled hydrophobic cleavage products preferentially assemble at the droplet-air interface at the surface of the sample droplet. In some embodiments, the fluorescence at the surface of the droplet is detected. In some embodiment, fluorescence at the surface of the droplet is an indicator of protease activity with minimal quenching by the sample.

### Lateral flow assay formats for protease detection assays

As an example not forming part of the claimed invention, provided herein is a lateral flow assay format for protease detection assays based on charge-changing substrates (Figure 6). In some embodiments, neutral or negatively charged substrates are incubated with a complex sample on a neutral section of a lateral flow surface. In some embodiments, the complex biological sample is selected from among blood, plasma, and serum. In some embodiment, the neutral incubation section is adjacent to a cationic section of the lateral flow surface. In some embodiments, the sample components and negatively charged cleavage products associate with the positively charged solid surface. In some embodiments, the positively charged cleavage products are fluorescently or colorimetrically labeled. In some embodiments, the fluorescently or colorimetrically labeled, positively charged cleavage product associates with the liquid phase of the lateral flow assay. In some embodiments, the cationic surface binds the negative components of blood. In some embodiments, the cationic surface binds negative components of blood and causes positive cleavage products to associate with the liquid phase more than the solid phase. In some embodiments, the cleavage product migrates further than the sample components. In some embodiments, the cleavage product migrates further than the negatively charged cleavage products. In some embodiments, the cleavage product migrates further than the sample components and the negatively charged cleavage products. In some embodiments, the migrating positively charged cleavage products are detected by their fluorescent label. In some embodiments, the migrating positively charged cleavage products are detected by their colorimetric label. In some embodiments, the fluorescent or colorimentric label is detected as a band away from the quenching sample. In some embodiments, the band indicates protease activity.

### Detection of non-degradative, charge-transferring enzymes

As an example not forming part of the claimed invention, provided herein is a method of detecting non-degradative, charge-transferring enzymes in an unprocessed sample. In some embodiments, the method uses charge-changing substrates and electrophoretic separation from an unprocessed sample. In some embodiments, the non-degradative enzymes are selected from phosphorylases and kinases. In some embodiments, substrates with phosphorylated residues are incubated with complex samples (Figure 7A). In some embodiments, the complex biological sample is selected from among blood, plasma, and serum. In some embodiments, the substrates with phosphorylated residues are selected from among serine, threonine, and tyrosine. In some embodiments, the substrates with phosphorylated residues are designed to become positively charged upon cleavage of the phosphate group. In some embodiments, cleavage of the phosphate group occurs by phosphorylases present in the sample. In some embodiments, the positively charged reaction product is separated from the complex sample. In some embodiments, the positively charged reaction product is separated from the complex sample by applying an electric field.

In some embodiments, positively charged substrates were incubated with complex samples. In some embodiments, the complex biological sample is selected from among blood, plasma, and serum. In some embodiments, the positively charged substrates contain residues selected from among serine, threonine, and tyrosine. In some embodiments, the substrates are designed to become negatively charged upon phosphorylation by kinases in the sample. In some embodiments, relative kinase activity across samples can be compared upon electrophoretic separation of phosphorylation products from the complex sample (Figure 7B). In some embodiments, kinases add a negatively charged phosphate group to a substrate. In some embodiments, greater kinase activity results in reduced fluorescence compared to samples with less kinase activity as phosphorylated substrates become negatively charged and separate in the direction of the complex sample rather than the positively charged non-phosphorylated substrate. In some embodiments, kinase activity is inferred by measuring the reduction in fluorescence at the band of non-phosphorylated substrate in an electrophoresis gel.

### AC/DC electrokinetic microarray devices for protease detection assays in blood

The ability to carry out the detection of proteases and other disease-related enzymes using AC/DC electrokinetic microarray devices would have special advantages for developing highly sensitive and specific assays in point of care (POC) and other formats. However, a general problem for electronic microarray devices is the relatively low levels of AC and DC voltages and currents that can be applied to devices reduces their effectiveness in high conductance (> 0.1 S/m) buffers and biological samples (blood, plasma, serum).

### AC/DC electrokinetic microarray devices

As an example not forming part of the claimed invention, provided herein are robust microarray devices that can be operated in both AC and DC modes. In some embodiments, each microelectrode is independently addressable by either direct micro-wiring or imbedded CMOS or other semiconductor control elements. In some embodiments, such devices are fabricated by photolithographic and other techniques well known for producing microelectronic devices. In some embodiments, microarrays are fabricated on silicon, glass, ceramic or plastic base substrate materials. In some embodiments, the microarrays are further insulated using silicon dioxide and silicon nitrate materials. In some embodiments, microelectrode and larger electrode structures are fabricated from platinum, palladium, gold, carbon, combinations thereof, or other conductor materials resistant to electrochemical degradation. In some embodiments, such microelectrodes and electrodes are fabricated onto the support structure using sputtering, e-beam or other deposition techniques well known to the microelectronics arts. In some embodiments, a key requirement is that the microelectrode structures is robust and resistant to electrochemical degradation. In some embodiments, microelectrodes are thicker (100 nm to 10 microns) and deposited under conditions which reduce defects and allow the microelectrodes/electrodes to function as a true solid metal. In some embodiments, microelectrodes on the array device have a variety of shapes which include, but are not limited to, circular, oval, rings, squares, rectangular and elongate wire like structures. In some embodiments, microelectrodes on the array device have a variety of sizes from 10 nm to 900 microns. In some embodiments, electrode structures range in size from 1 mm to 10 cm. In some embodiments, the number of microelectrodes on the array ranges from two to twenty thousand with spacing that ranges from 100 nm to 1 mm. In some embodiments, the microelectrode array structure is over coated with porous structure or permeation layer. In some embodiments this allows the sample solution (electrolyte, cations and anions) to contact the metal electrode surface, but prevents the larger biomolecules from reaching the surface where they can be damaged by the electrolysis products (H⁺, OH⁻, O₂, H₂, free radicals, heat, bubbles, etc.). Such permeation layers could include but are not limited to hydrogels (agarose, polyacrylamide, etc.), glass, organic, polymer, nylon net-like or porous structures; as well as lawns of nanostructures which achieve the desired permeation layer properties. In some embodiments, permeation layers range in thickness from 10 nm to 500 microns. In some embodiments, the permeation layer is impregnated with covalent and non-covalent binding agents. In some embodiments, the binding agents allow attachment of other binding agents. Such binding agents include, but are not limited to, amines, thiol groups, carboxyl groups, biotin, streptavidin, antibodies, aptamers, oligonucleotides, etc. In some embodiments, the binding agents are in specific areas of the microarray (on specific microelectrodes). In some embodiments, different binding agents are located in different areas of the microarray. In some embodiments, electrokinetic microarrays (chips) are enclosed in a sample chamber structure with associated microfluidics that allow samples, wash buffers and reagents to be delivered in a controlled manner. In some embodiments, the sample chamber structure has a component that secures the microarray (chip), prevents contact with the electrolyte and provides outside electrical connection to the microarray for providing the AC and DC voltages and currents required to carry out AC dielectrophoresis (DEP) and AC electrophoresis processes. In some embodiments, the sample chamber structure also has a glass, silica or plastic window over the microarray (chip) which would allow visual monitoring as well as color and/or epifluorescent detection. In some embodiments, the microarray (chip) enclosed in fluid sample chamber is connected to an AC/DC power supply and function generator; associated fluidic components for delivering samples, wash buffers and reagents and for accepting eluted samples and fluidic waste; visual monitoring and epifluorescent microscope and associated computer for overall control, chip monitoring, detection and analysis. In some embodiments, all components constitute a complete Protease Assay Detection System.

### Microarray protease assays carried out in AC mode

In the case of operating even a robust microarray device in the AC mode for dielectrophoretic (DEP) separations in blood, effective working voltages are limited to <25 volts AC before aggressive electrochemistry begins to disrupts the process. Under these conditions (<25 volts AC) red and white cells are concentrated into the DEP high field regions (between the microelectrodes), nanoscale entities (20 nm-800 nm) are concentrated into DEP high field regions (on the microelectrodes), while the smaller biomolecules remain relatively unaffected by the DEP fields. After the separation, a fluid wash can be used to remove the cells and other low molecular weight plasma components from the microarray device, while the nanoscale entities remain concentrated in the DEP high field areas.

In some embodiments, a protease assay on a microarray device operated in the AC mode utilizes special nanoparticle entities along with the charge changing peptide substrates which have affinity for the charge changing peptide products. In some embodiments, as the peptide substrates are cleaved by the protease in the blood, the charge changing peptide products become bound to the special nanoparticle entities. In some embodiments, these nanoparticle entities include, but are not limited to, fluorescent and non-fluorescent nanoparticles in size ranges from 20 nm to 800 nm; polymer, lipid based, metallic, semiconductor, and ceramic nanoparticles; nanoparticles with negative or positive charges, nanoparticles modified with ligand binding agents for covalent and noncovalent binding, including antibodies, aptamers and lectins. In some embodiments, after the protease reaction is complete, the AC field is applied and the nanoparticles with the bound peptide products are concentrated into the DEP high field areas on the microelectrodes, while blood cells are concentrated into the low field areas. In some embodiments, a fluid wash is then applied. In some embodiments, the fluid wash removes the blood cells and plasma components along with any unreacted charge changing peptide substrate. In some embodiments, the nanoparticles-peptide products remain concentrated in the DEP high field area where they are detected.

In some embodiments, specific protease assays are carried out using negatively charged fluorescent charge changing peptide substrates that produce positively charged fluorescent peptide products together with negatively charged non-fluorescent nanoparticles. In some embodiments, upon cleavage by the specific protease in the blood, the positively charged fluorescent peptide product binds to the negatively charged non-fluorescent nanoparticles. In some embodiments, the AC field is applied and the nanoparticles with bound fluorescent charge changing peptide product are isolated into the DEP high field regions. In some embodiments, a fluidic wash is then used to remove the cells, plasma materials, and any un-reacted negatively charged fluorescent charge changing peptide substrate. In some embodiments, the nanoparticle-fluorescent peptide product remains isolated on the microelectrodes for epifluorescent detection and analysis. This example involving a negatively charged fluorescent charge changing peptide with negatively charged non-fluorescent nanoparticles is shown in Figure 7. Many other such AC/DC microarray assay formats are within the scope of this invention, one further example would include using non-fluorescent nanoparticles labelled with antibodies which are specific for the charge changing peptide products. Many other AC mode assay formats are possible.

### Microarray protease assays carried out in DC mode

In the case of operating even a robust microarray device in the DC mode for electrophoretic based separations in blood, effective working voltages are limited to <10 volts DC before aggressive electrochemistry begins to disrupts the process. An additional problem for DC mode operations is that the electrophoretic attraction of charged entities like the peptide substrates and products is greatly reduced in high conductance (>0.5 S/m) buffers and biological samples (blood, plasma, serum).

In some embodiments, provided are novel methods and procedures that allow protease assays and detection of charge-changing peptide products to be carried out by microarray devices operated in the DC mode. By way of just one of many examples, a blood sample with a specific negatively charged fluorescent charge changing peptide substrate would be applied to the microarray chip device. After the appropriate reaction time, a DC field would be applied to the chip with a group of microelectrodes near to the fluidic input port biased negative and a group of microelectrodes near to the outlet port biased positive. At the same time, a wash solution containing a low conductance zwitterionic buffer would be slowly delivered to the chip through the inlet port. As the low conductance buffer moves over the chip the positively charged fluorescent charge changing peptide products will become more electrophoretically attracted to the negatively charged microelectrodes on one side of the chip device while all other components, cells, plasma proteins and negatively charged fluorescent charge changing peptide substrates are electrophoretically attracted to the opposite side of the microarray. Ultimately, the flow of the wash fluid will remove most of the cells and plasma proteins, while the positively charged fluorescent charge changing peptide products remain concentrated on the set of microelectrodes near the input port. At this point the fluorescent peptide products can be detected and analyzed by epifluorescence. In this procedure, low conductance buffers can include but are not limited to zwitterionic histidine buffer in the 1 mM to 100 mM range. Many other DC mode separation formats and detection schemes are well within the scope of this invention.

### EXAMPLES

### Example 1: Identifying the proteolytic signature of complex samples

A synthetic peptide library containing combinations of amino acid pairs, combinations of positively and negatively charged residues or modified residues at different positions along the length of the synthetic peptides, and a tag such as a fluorescent dye was prepared (Figure 1A). Substrate signatures were generated after an incubation of complex samples (e.g., blood, plasma, serum) with the substrate library and separation of the cleavage products of the incubated substrate library from the complex sample by gel electrophoresis, capillary electrophoresis, or a combination of DC and AC electrokinetic techniques.

Individual substrates in the peptide mixture were ranked according to migration and detected via fluorescence or other tag. To dissect the contribution of multiple classes of protease activity such as serine, cysteine, and metallopeptidases, samples were tested after being incubated separately with various activity inhibitors such as the metal chelator EDTA, the cysteine peptidase inhibitors E-64 and CAO74, or the elastase-specific chloromethyl ketone inhibitor. Cleavage-site specificity data from inhibited samples was combined to generate a substrate signature for each peptidase class. Secondary synthetic peptide libraries were generated to further define the cleavage preferences of peptidases in complex samples.

### Example 2: In vitro / in vivo purification by activity based probes

An ABP composed of a chemically reactive group attached to a positively charged moiety via a spacer molecule was designed (Figures 2A-2D). The ABP was incubated with a sample *in vitro* or injected *in vivo.* Upon binding of the ABP to the protease active site, the chemically reactive group reacted irreversibly with the active site nucleophilic residue. The positively charged moiety of the ABP remained exposed outside the active site of the protease. Electrophoretic separation *in vitro* was used to separate the proteases that have bound ABP since the positively charged moiety separated from the majority of components of the complex biological sample that were negatively charged. Following *in vivo* injection of an ABP with a positively charged moiety and an identifying tag such as a near-infrared or fluorescent dye, visualization of protease activity was possible, such as at the site of a tumor which was biopsied. After biopsy the ABP-bound proteases were separated from the complex sample by electrophoretic separation. ABP-bound proteases that had been separated from complex samples were available for further downstream applications and characterization of post-translational modifications, mutations, etc.

### Example 3: In vivo protease detection by peptides

A negatively charged fluorescently labeled peptide was injected into a mouse. Upon encountering the acidic microenvironment of a tumor, the histidine residues of the peptide became positively charged and the peptide was overall neutral, which allowed the peptide to penetrate the tumor. The peptide was cleaved by the protease for which its sequence was designed. The positively charged, fluorescent or near-infrared labeled cleavage product stuck to the cell surface and allowed *in vivo* imaging of protease activity and localization (Figures 3A-3D).

### Example 4: In vivo protease detection by peptide decorated nanoparticles

A nanoparticle decorated with negatively charged peptides was injected into a mouse. Upon encountering the acidic microenvironment of a tumor, the histidine residues of the peptide became positively charged and the peptide was overall neutral, which allowed the decorated nanoparticle to penetrate the tumor. The peptide on the surface of the nanoparticle was cleaved by the protease for which its sequence was designed. The cleavage product was positively charged which caused the decorated nanoparticle to stick to the cell surface and release its payload of imaging agents and/or drug molecules into the tumor cell (Figures 4A-4E).

### Example 5: Detection of amphiphilic enzyme substrates using contact angle measurement

A technique to detect protease activity in droplets of complex samples by contact angle measurement was developed (Figure 5A). Amphiphilic substrates were designed that contained a hydrophobic and hydrophilic section around a cleavage site for a protease of interest. The substrates were incubated with a droplet of a complex sample (e.g., blood) and as proteolytic cleavage occurred, hydrophilic and hydrophobic products were released from the amphiphilic substrate. The hydrophobic cleavage products preferentially assembled at the droplet-air interface at the surface of the sample droplet. As more hydrophobic cleavage products migrated to the surface of the droplet, the contact angle of the droplet decreased and was measured as an indicator of protease activity.

### Example 6: Detection of amphiphilic enzyme substrates on droplet surfaces

A technique to detect protease activity in droplets of complex samples by fluorescence detection at the surface of the droplet was developed (Figure 5B). Fluorescently labeled amphiphilic substrates were designed to contain a hydrophobic and hydrophilic section around a cleavage site for a protease of interest. The substrates were incubated with a droplet of a complex sample (e.g., blood), and as proteolytic cleavage occurred, hydrophilic and hydrophobic products were released from the amphiphilic substrate. The fluorescently labeled hydrophobic cleavage products preferentially assembled at the droplet-air interface at the surface of the sample droplet. The fluorescence at the surface of the droplet was detected as an indicator of protease activity with minimal quenching by the sample.

### Example 7: Lateral flow assays of charge-changing substrates

A lateral flow assay format for protease detection assays based on charge-changing substrates was developed (Figure 6). Neutral or negatively charged substrates were incubated with complex sample on a neutral section of a lateral flow surface. The neutral incubation section was adjacent to a cationic section of the lateral flow surface that caused sample components and negatively charged cleavage products to associate with the positively charged solid surface. The fluorescently or colorimetrically labeled positively charged cleavage product associated with the liquid phase of the lateral flow assay and migrated further than the sample components or the negatively charged cleavage products. The migrating positively charged cleavage products were detected by their fluorescent or colorimetric label as a band away from the quenching sample which indicated protease activity.

### Example 8: Detection of non-degradative, charge-transferring enzymes

A method by which to measure non-degradative, charge-transferring enzymes such as kinases and phosphorylases based on charge-changing substrates and electrophoretic separation from complex samples was developed. Substrates with phosphorylated residues such as serine, threonine, and tyrosine, were incubated with complex samples and were designed to become positively charged upon cleavage of the phosphate group by phosphorylases present in the sample (Figure 7A). The positively charged reaction product was then separated from the complex sample by applying an electric field.

Positively charged substrates containing residues such as serine, threonine, and tyrosine, were incubated with complex samples and were designed to become negatively charged upon phosphorylation by kinases in the sample (Figure 7B). Relative kinase activity across samples was compared upon electrophoretic separation of phosphorylation products from the complex sample. Greater kinase activity resulted in reduced fluorescence compared to samples with less kinase activity as phosphorylated substrates became negatively charged and separated in the direction of the complex sample rather than the positively charged non-phosphorylated substrate. Kinase activity was inferred by measuring the reduction in fluorescence at the band of non-phosphorylated substrate in an electrophoresis gel.

### Example 9: Thrombin detection using a charge-changing peptide

Various concentrations (0 nM, 0.5 nM, 5 nM, 10 nM, 20 nM, or 100 nM) of the thrombin targeted charge-changing peptide shown in Figure 9 (and with the sequence Ac-N-D⁻-D⁻-Nle-T-P-R⁺//G-S-A-G-A-G-A-G-diamino-ethyl-BFL) was incubated in either 1xPBS (Figure 10A) or citrated blood (Figure 10B), both of which contained thrombin. After incubation, fluorescence was measured, indicating specific binding of the peptide to the thrombin in the sample. Fluorescence increased with increasing thrombin concentration.

### Example 10: Whole blood thrombin detection

The thrombin targeted charge-changing peptide shown in Figure 9 (and with the sequence Ac-N-D⁻-D⁻-Nle-T-P-R⁺//G-S-A-G-A-G-A-G-diamino-ethyl-BFL) was incubated in untreated whole blood for up to 33 minutes. A negative control was also prepared See Figure 11A. Fluorescence was measured at 3 minute intervals during incubation. Fluorescence signal, indicating specific binding of the peptide to thrombin in the whole blood sample, increased over time, with peak values observed after about 18 minutes (Figure 11B).

### Example 11: Cathepsin-S detection in buffer

The cathepsin-S targeted charge-changing peptide shown in Figure 9 (and with the sequence N-SUC⁻-E⁻-G-R⁺-W-H-T-V-G//L-R⁺-W-E⁻-C(Cy5⁻)-R⁺-CO-NH₂) was incubated in 1xPBS buffer with varying concentrations of cathepsin-S. After incubation, fluorescence was measured, indicating specific binding of the peptide to the cathepsin-S in the sample. Fluorescence increased with increasing cathepsin-S concentration (Figure 12).

### Example 12: Coagulation monitoring

To detect thrombin activity and monitor coagulation, the thrombin targeted charge-changing peptide shown in Figure 9 (and with the sequence of Ac-N-D⁻-D⁻-Nle-T-P-R⁺//G-S-A-G-A-G-A-G-diamino-ethyl-BFL) was incubated in a drop of whole blood from subjects taking an anticoagulant (aspirin) and from control subjects that were not taking an anticoagulant. Fluorescence signal, indicating specific binding of the peptide to thrombin in the whole blood sample, was measured at various time points up to 30 minutes. The top panel of Figure 13A shows fluorescence in the control group while the bottom panel shows fluorescence in the anticoagulant group. Fluorescence increased in both subject groups over time, but more quickly in the control group (Figure 13B).

### Example 13: Protease (trypsin) activity measurements using an electrophoresis focusing assay and miniaturized array prototype

A miniaturized array prototype was used to measure trypsin activity using an electrophoresis focusing assay (Figure 15A). A charge-changing trypsin-specific peptide with a fluorescent tag was incubated with sample containing various concentrations of trypsin. Electrophoretic separation was used to separate trypsin bond to a charge-changing peptide substrate. The trypsin with bound substrate were positively charged and separated from the majority of components of the complex biological sample that were negatively charged. Increased fluorescence was observed with an increase in concentration of trypsin (Figure 15B).

Trypsin activity was measured after one week using a miniaturized array prototype (Figure 16A). Increased fluorescence was observed with an increase in concentration of trypsin (Figure 16B).

Trypsin activity was also measured in a blood sample using a miniaturized array prototype (Figure 17A). Increased fluorescence was observed with an increase in concentration of trypsin (Figure 17B).

### Example 14: Concentration and detection of proteases using DEP

Dielectrophoretic (DEP) separation was used to detect and determine concentration of MMP2 and MMP9 in plasma samples from chronic lymphocytic leukemia subjects and compared to control subjects. Plasma samples were incubated with a charge-changing peptide substrate specific for MMP2/9. An AC field was applied to some of the plasma samples and the proteases with bound fluorescent charge changing peptide product were isolated into the DEP high field regions. As shown in Figure 19, fluorescence intensity was greater in all subjects in the DEP plasma samples (black bars) as compared to plasma samples not subjected to DEP (white bars).

### REFERENCES

**Biological significance of proteolytic and post-translational modifications**
   Barrett, A. J. (2004) Bioinformatics of proteases in the MEROPS database. Curr. Opin. Drug. Discov. Dev. 7, 334-341.
   Cohen, P. (2002) The origins of protein phosphorylation. Nat. Cell Biol. 4, E127-E130.
   Daviet, L., and Colland, F. (2008) Targeting ubiquitin specific proteases for drug discovery. Biochimie (Paris) 90, 270-283.
   Geiss-Friedlander, R., and Melchior, F. (2007) Concepts in sumoylation: a decade on. Nat. Rev. Mol. Cell Biol. 8, 947-956.
   Nandi, D., Tahiliani, P., Kumar, A., and Chandu, D. (2006) The ubiquitin-proteasome system. J. Biosci. 31, 137-155.
   Polevoda, B., and Sherman, F. (2003) N-terminal acetyltransferases and sequence requirements for N-terminal acetylation of eukaryotic proteins. J. Mol. Biol. 325, 595-622.
   Puente, X. S., Sanchez, L. M., Overall, C. M., and López-Otín, C. (2003) Human and mouse proteases: a comparative genomic approach. Nat. Rev. Genet. 4, 544-558.
   Sadoul, K., Boyault, C., Pabion, M., and Khochbin, S. (2008) Regulation of protein turnover by acetyltransferases and deacetylases. Biochimie (Paris) 90, 306-312.
   Walsh, G., and Jefferis, R. (2006) Post-translational modifications in the context of therapeutic proteins. Nat. Biotechnol. 24, 1241-1252.
**Diseases and therapy targeting of proteases and post-translational enzymes**
   Coussens, L. M., Fingleton, B., Matrisian, L. M. (2002) Matrix metalloproteinase inhibitors and cancer: trials and tribulations. Science 295, 2387-2392.
   Drag, M., and Salvesen, G. S. (2010) Emerging principles in protease-based drug discovery. Nat. Rev. Drug Discovery 9, 690-701.
   Gutierrez-Fernandez, A., Fueyo, A., Folgueras, A. R., Garabaya, C., Pennington, C. J., Pilgrim, S., Edwards, D. R., Holliday, D. L., Jones, J. L., Span, P. N., Sweep, F. C., Puente, X. S., and López-Otín, C. (2008) Matrix metalloproteinase-8 functions as a metastasis suppressor through modulation of tumor cell adhesion and invasion. Cancer Res. 68, 2755-2763.
   Hegedus, L., Cho, H., Xie, X., and Eliceiri, G. L. (2008) Additional MDA-MB-231 breast cancer cell matrix metalloproteinases promote invasiveness. J. Cell. Physiol. 216, 480-485.
   McQuibban, G. A., Gong, J. H., Tam, E. M., McCulloch, C. A., Clark-Lewis, I., and Overall, C. M. (2000) Inflammation dampened by gelatinase A cleavage of monocyte chemoattractant protein-3. Science 289, 1202-1206.
   McQuibban, G. A., Gong, J. H., Wong, J. P., Wallace, J. L., Clark-Lewis, I., and Overall, C. M. (2002) Matrix metalloproteinase processing of monocyte chemoattractant proteins generates CC chemokine receptor antagonists with anti-inflammatory properties in vivo. Blood 100, 1160-1167.
   Overall, C. M., and Kleifeld, O. (2006) Towards third generation matrix metalloproteinase inhibitors for cancer therapy. Br. J. Cancer 94, 941-946.
   Overall, C. M., and Kleifeld, O. (2006) Tumour microenvironment - opinion: validating matrix metalloproteinases as drug targets and anti-targets for cancer therapy. Nat. Rev. Cancer 6, 227-239.
   Stoch, S. A., and Wagner, J. A. (2008) Cathepsin K inhibitors: a novel target for osteoporosis therapy. Clin. Pharmacol. Ther. 83, 172-176.
   Tremblay, G. M., Janelle, M. F., and Bourbonnais, Y. (2003) Anti-inflammatory activity of neutrophil elastase inhibitors. Curr. Opin. Investig. Drugs 4, 556-565.
   Turk, B. (2006) Targeting proteases: successes, failures and future prospects. Nat. Rev. Drug Discov. 5, 785-799.
   Yang, X., Dong, Y., Zhao, J., Sun, H., Deng, Y., Fan, J., and Yan, Q. (2007) Increased expression of human macrophage metalloelastase (MMP-12) is associated with the invasion of endometrial adenocarcinoma. Pathol. Res. Pract. 203, 499-505.
**Microarray technologies for degradomics**
   Chen, G. Y., Uttamchandani, M., Zhu, Q., Wang, G., Yao, S. Q. (2003) Developing a strategy for activity-based detection of enzymes in a protein microarray. ChemBioChem 4, 336-339.
   Eppinger, J., Funeriu, D. P., Miyake, M., Denizot, L., Miyake, J. (2004) Enzyme Microarrays: On-Chip Determination of Inhibition Constants Based on Affinity-Label Detection of Enzymatic Activity. Angew. Chem. 116, 3894-3898.
   Funeriu, D. P., Eppinger, J., Denizot, L., Miyake, M., Miyake, J. (2005) Enzyme family-specific and activity-based screening of chemical libraries using enzyme microarrays. Nat. Biotechnol. 23, 622-627.
   Overall, C. M., Tam, E. M., Kappelhoff, R., Connor, A., Ewart, T., Morrison, C. J., Puente, X., López-Otín, C., and Seth, A. (2004) Protease degradomics: mass spectrometry discovery of protease substrates and the CLIP-CHIP, a dedicated DNA microarray of all human proteases and inhibitors. Biol. Chem. 385, 493-504.
   Pease, A. C., Solas, D., Sullivan, E. J., Cronin, M. T., Holmes, C. P., Fodor, S. P. (1994) Light-generated oligonucleotide arrays for rapid DNA sequence analysis. Proc. Natl. Acad. Sci. USA 91, 5022-5026.
   Sieber, S. A., Mondala, T. S., Head, S. R., Cravatt, B. F. (2004) Microarray platform for profiling enzyme activities in complex proteomes. J. Am. Chem. Soc. 126, 15640-15641.
   Uttamchandani, M., Liu, K., Panicker, R. C., Yao, S. Q. (2007) Activity-based fingerprinting and inhibitor discovery of cysteine proteases in a microarray. Chem. Commun. 1518-1520*.*
   Winssinger, N., Damoiseaux, R., Tully, D. C., Geierstanger, B. H., Burdick, K., Harris, J. L. (2004) PNA-encoded protease substrate microarrays. Chem. Biol. 11, 1351-1360.
   Wu, H., Ge, J., Yang, P.-Y., Wang, J., Uttamchandani, M., Yao, S. Q. (2011) A peptide aldehyde microarray for high-throughput profiling of cellular events. J. Am. Chem. Soc. 133, 1946-1954.
**Mass spectrometry technologies for degradomics**
   Aebersold, R., and Mann, M. (2003) Mass spectrometry-based proteomics. Nature 422, 198-207.
   Dean, R.A. and Overall, C.M. (2007) Proteomics discovery of metalloproteinase substrates in the cellular context by iTRAQ labeling reveals a diverse MMP-2 substrate degradome. Mol. Cell Proteomics 6, 611-623.
   Domon, B., and Aebersold, R. (2006) Mass spectrometry and protein analysis. Science 312, 212-217.
   López-Otín, C., and Overall, C. M. (2002) Protease degradomics: a new challenge for proteomics. Nat. Rev. Mol. Cell Biol. 3, 509-519.
   Mann, M., Hendrickson, R. C., and Pandey, A. (2001) Analysis of proteins and proteomes by mass spectrometry. Annu. Rev. Biochem. 70, 437-473.
   O'Donoghue, A. J., Eroy-Reveles, A. A., Knudsen, G. M., Ingram, J., Zhou, M., Statnekov, J. B., ... Craik, C. S. (2012) Global identification of peptidase specificity by multiplex substrate profiling. Nature Methods, 9(11), 1095-1100.
   Overall, C. M., Tam, E. M., Kappelhoff, R., Connor, A., Ewart, T., Morrison, C. J., Puente, X., López-Otín, C., and Seth, A. (2004) Protease degradomics: mass spectrometry discovery of protease substrates and the CLIP-CHIP, a dedicated DNA microarray of all human proteases and inhibitors. Biol. Chem. 385, 493-504.
   Schwartz, D. R., Moin, K., Yao, B., Matrisian, L. M., Coussens, L. M., Bugge, T. H., Fingleton, B., Acuff, H. B., Sinnamon, M., Nassar, H., Platts, A. E., Krawetz, S. A., Linebaugh, B. E., and Sloane, B. F. (2007) Hu/Mu ProtIn oligonucleotide microarray: dual-species array for profiling protease and protease inhibitor gene expression in tumors and their microenvironment. Mol. Cancer Res. 5, 443-54.
**Activity-based probes and mass spectrometry analysis for degradomics**
   Blum, G., von Degenfeld, G., Merchant, M. J., Blau, H. M., and Bogyo, M. (2007) Noninvasive optical imaging of cysteine protease activity using fluorescently quenched activity-based probes. Nat. Chem. Biol. 3, 668- 677
   Chan, E. W., Chattopadhaya, S., Panicker, R. C., Huang, X., and Yao, S. Q. (2004) Developing photoactive affinity probes for proteomic profiling: hydroxamate-based probes for metalloproteases. J. Am. Chem. Soc. 126, 14435- 14446.
   Chun, J., Yin, Y. I., Yang, G., Tarassishin, L., and Li, Y. M. (2004) Stereoselective synthesis of photoreactive peptidomimetic γ-secretase inhibitors. J. Org. Chem. 69, 7344-7347.
   Grabarek, J., and Darzynkiewicz, Z. (2002) In situ activation of caspases and serine proteases during apoptosis detected by affinity labeling their enzyme active centers with fluorochrome-tagged inhibitors. Exp. Hematol. 30, 982- 989.
   Greenbaum, D., Medzihradszky, K. F., Burlingame, A., and Bogyo, M. (2000) Epoxide electrophiles as activity-dependent cysteine protease profiling and discovery tools. Chem. Biol. 7, 569- 581.
   Greenbaum, D., Baruch, A., Hayrapetian, L., Darula, Z., Burlingame, A., Medzihradszky, K. F., and Bogyo, M. (2002) Chemical approaches for functionally probing the proteome. Mol. Cell. Proteomics 1, 60- 68.
   Kessler, B. M., Tortorella, D., Altun, M., Kisselev, A. F., Fiebiger, E., Hekking, B. G., Ploegh, H. L., and Overkleeft, H. S. (2001) Extended peptide-based inhibitors efficiently target the proteasome and reveal overlapping specificities of the catalytic beta-subunits. Chem. Biol. 8, 913- 929.
   Kidd, D., Liu, Y., and Cravatt, B. F. (2001) Profiling serine hydrolase activities in complex proteomes. Biochemistry 40, 4005- 4015.
   Liu, Y., Patricelli, M. P., and Cravatt, B. F. (1999) Activity-based protein profiling: the serine hydrolases. Proc. Natl. Acad. Sci. U. S. A. 96, 14694- 14699.
   Saghatelian, A., Jessani, N., Joseph, A., Humphrey, M., and Cravatt, B. F. (2004) Activity-based probes for the proteomic profiling of metalloproteases. Proc. Natl. Acad. Sci. U. S. A. 101, 10000- 10005.
   Schmidinger, H., Hermetter, A., and Birner-Gruenberger, R. (2006) Activity-based proteomics: enzymatic activity profiling in complex proteomes. Amino Acids 30, 333- 350.
   Thornberry, N. A., Peterson, E. P., Zhao, J. J., Howard, A. D., Griffin, P. R., and Chapman, K. T. (1994) Inactivation of interleukin-1β converting enzyme by peptide (acyloxy)methyl ketones. Biochemistry 33, 3934- 3940.
   Wang, C. C., Bozdech, Z., Liu, C. L., Shipway, A., Backes, B. J., Harris, J. L., and Bogyo, M. (2003) Biochemical analysis of the 20 S proteasome of Trypanosoma brucei. J. Biol. Chem. 278, 15800- 15808.
   Williams, E. B., Krishnaswamy, S., and Mann, K. G. (1989) Zymogen/enzyme discrimination using peptide chloromethyl ketones. J. Biol. Chem. 264, 7536-7545.
**Challenge of differentiating between active versus inactive forms of enzymes**
   Moldoveanu, T., Hosfield, C. M., Lim, D., Elce, J. S., Jia, Z. C., Davies, P. L. (2002) A Ca(2+) switch aligns the active site of calpain. Cell 108, 649-660.
   Turk, V., Turk, B., Turk, D. (2001) Lysosomal cysteine proteases: facts and opportunities. EMBO J. 20, 4629-4633.
**Zymography and other reverse degradomics technologies**
   Baragi, V.M. et al. (2000) A versatile assay for gelatinases using succinylated gelatin. Matrix Biol. 19, 267-273.
   Kaberdin, V.R. and McDowall, K.J. (2003) Expanding the use of zymography by the chemical linkage of small, defined substrates to the gel matrix. Genome Res. 13, 1961-1965.
   Kleiner, D.E. and Stetler-Stevenson, W.G. (1994) Quantitative zymography: detection of picogram quantities of gelatinases. Anal. Biochem. 218, 325-329.
   Kupai, K. et al. (2010) Matrix metalloproteinase activity assays: importance of zymography. J. Pharmacol. Toxicol. Methods 61, 205-209.
   McKerrow, J.H., Pino-Heiss, S., Lindquist, R. and Werb, Z. (1985) Purification and characterization of an elastinolytic proteinase secreted by cercariae of Schistosoma mansoni. J. Biol. Chem.260, 3703-3707.
   Paemen, L. et al. (1996) The gelatinase inhibitory activity of tetracyclines and chemically modified tetracycline analogues as measured by a novel microtiter assay for inhibitors. Biochem. Pharmacol. 52, 105-111.
   Piccard, H. et al. (2009) "Reverse degradomics", monitoring of proteolytic trimming by multi-CE and confocal detection of fluorescent substrates and reaction products. Electrophoresis 30, 2366-2377.
   Vandooren, J. et al. (2011) Gelatin degradation assay reveals MMP-9 inhibitors and function of O-glycosylated domain. World J. Biol. Chem. 2, 14-24.
   Wilkesman, J. and Kurz, L. (2009) Protease analysis by zymography: a review on techniques and patents. Recent Pat. Biotechnol. 3, 175-184.
***In vivo* imaging of enzyme activity**
   Bremer, C., Tung, C.H. & Weissleder, R. In vivo molecular target assessment of matrix metalloproteinase inhibition. Nat. Med. 7, 743-748 (2001).
   Crawford, B.D. & Pilgrim, D.B. Ontogeny and regulation of matrix metalloproteinase activity in the zebrafish embryo by in vitro and in vivo zymography. Dev. Biol. 286, 405-414 (2005).
   Kaijzel, E.L., van der Pluijm, G. & Lowik, C.W. Whole-body optical imaging in animal models to assess cancer development and progression. Clin. Cancer Res. 13, 3490-3497(2007).
   Keow, J.Y., Pond, E.D., Cisar, J.S., Cravatt, B.F. & Crawford, B.D. Activity-based labeling of matrix metalloproteinases in living vertebrate embryos. PLoS ONE 7, e43434 (2012).
   Saghatelian, A., Jessani, N., Joseph, A., Humphrey, M. & Cravatt, B.F. Activity-based probes for the proteomic profiling of metalloproteases. Proc. Natl. Acad. Sci. USA 101,10000-10005 (2004).
   Scherer, R.L., VanSaun, M.N., McIntyre, J.O. & Matrisian, L.M. Optical imaging of matrix metalloproteinase-7 activity in vivo using a proteolytic nanobeacon. Mol. Imaging 7,118-131 (2008).
   Weissleder, R., Tung, C.H., Mahmood, U. & Bogdanov, A. Jr. In vivo imaging of tumors with protease-activated near-infrared fluorescent probes. Nat. Biotechnol. 17, 375-378 (1999).
   Wyatt, R.A. et al. The zebrafish embryo: a powerful model system for investigating matrix remodeling. Zebrafish 6, 347-354 (2009).

## Claims

1. A method of identifying protease activity in a complex biological sample selected from blood, plasma, and serum, the method comprising:
a) incubating the complex sample with a synthetic peptide library to generate cleavage products, wherein the synthetic peptide library comprises combinations of amino acid pairs, positively and negatively charged residues, or modified residues at different positions along the length of the synthetic peptides, and a fluorescent tag;
b) separating the cleavage products from the complex sample; and
c) generating a proteolytic signature for the complex sample; wherein the synthetic peptide library comprises synthetic charge-changing fluorescent peptide substrates specific for proteases, wherein the charge-changing peptide sequences selectively target proteases selected from coagulation related proteases, metalloproteases, cathepsins, bacterial proteases, kallikreins, and calpains,
wherein the coagulation related protease is thrombin and the charge-changing peptide sequence has the sequence Ac-N-D-D-Nle-T-P-R-G-S-A-G-A-G-A-G-diamino-ethyl-Bodipy FL (BFL),
wherein the metalloprotease is MMP1 and the charge-changing peptide sequence has the sequence Ac-N-D-G-P-Q-A-I-A-G-Q-G-A-G-diamino-ethyl-BFL,
wherein the metalloprotease is MMP3 and the charge-changing peptide sequence has the sequence Ac-D-G-P-K-P-V-E-Nva-Y-N-K(ε-BFL)-NH₂,
wherein the metalloprotease is MMP8 and the charge-changing peptide sequence has the sequence set forth in Ac-N-D-G-P-Q-G-Y-A-G-Q-G-A-G-diamino-ethyl-BFL,
wherein the cathepsin is cathepsin-S and the charge-changing peptide sequence has the sequence N-SUC-E-G-R-W-H-T-V-G-L-R-W-E-C(Cy5)-R-CO-NH₂,
wherein the cathepsin is cathepsin-D and the charge-changing peptide sequence has the sequence N-SUC-D-D-L-V-V-L-F-V-K-K-C(Cy5)-A-CO-NH₂,
wherein the bacterial protease is Omp-T and the charge-changing peptide sequence has the sequence Ac-SUC-D-G-D-K-Y-R-R-A-W-G-D-T-I-diamino-ethyl-BFL,
wherein the bacterial protease is SspB and the charge-changing peptide sequence has the sequence Ac-D-G-D-A-F-S-K-A-L-P-K(ε-BFL)-NH₂,
wherein the kallikrein is kallikrein 2 and the charge-changing peptide sequence has the sequence Ac-N-D-G-D-T-F-R-S-A-A-G-K(ε-BFL)-NH₂,
wherein the kallikrein is kallikrein 3 and the charge-changing peptide sequence has the sequence Ac-N-D-G-S-S-I-Y-Q-S-S-T-G-diamino-ethyl-BFL, or
wherein the calpain is calpain 1 or calpain 2 and the charge-changing peptide sequence has the sequence N-SUC-E-P-L-F-A-A-R-K(ε-BFL)-NH₂.

2. The method of claim 1, wherein the fluorescent tag is a fluorescent dye.

3. The method of claim 1 or 2, wherein the cleavage products are separated from the complex sample by gel electrophoresis, capillary electrophoresis, or a combination of DC and AC electrokinetic techniques.

4. The method of any one of claims 1-3, wherein the proteolytic signature is generated by detecting and ranking individual substrates in the peptide mixture according to migration.

5. The method of claim 4, wherein the individual substrates are detected via the tag.

6. The method of any one of claims 1-5, wherein the proteolytic signature comprises multiple classes of protease activity.

7. The method of claim 6, wherein the multiple classes of protease activity are selected from serine, cysteine, threonine, aspartyl, and metallopeptidase activity.

8. The method of claim 6, wherein the multiple classes of protease activity are detected by separately incubating the complex sample with an activity inhibitor.

9. The method of claim 8, wherein the activity inhibitor is selected from a metal chelator, a cysteine peptidase inhibitor, and an elastase-specific inhibitor.

10. The method of claim 9, wherein the activity inhibitor is selected from EDTA, E-64, CAO74, and a chloromethyl ketone inhibitor.

## Patentansprüche

1. Verfahren zum Identifizieren von Proteaseaktivität in einer komplexen biologischen Probe, die aus Blut, Plasma und Serum ausgewählt ist, wobei das Verfahren Folgendes umfasst:
a) Inkubieren der komplexen Probe mit einer synthetischen Peptidbibliothek zur Erzeugung von Spaltprodukten, wobei die synthetische Peptidbibliothek Kombinationen von Aminosäurepaaren, positiv und negativ geladenen Resten oder modifizierten Resten an verschiedenen Positionen über die gesamte Länge der synthetischen Peptide umfasst, und einem Fluoreszenz-Tag;
b) Trennen der Spaltprodukte von der komplexen Probe; und
c) Erzeugen einer proteolytischen Signatur für die komplexe Probe; wobei die synthetische Peptidbibliothek synthetische ladungsändernde fluoreszierende Peptidsubstrate umfasst, die für Proteasen spezifisch sind, wobei die ladungsändernden Peptidsequenzen selektiv auf Proteasen zielen, die aus gerinnungsverbundenen Proteasen, Metalloproteasen, Cathepsinen, bakteriellen Proteasen, Kallikreinen und Calpainen ausgewählt sind,
wobei es sich bei der gerinnungsverbundenen Protease um Thrombin handelt und die ladungsändernde Peptidsequenz die Sequenz Ac-N-D-D-Nle-T-P-R-G-S-A-G-A-G-A-G-Diaminoethyl-Bodipy FL (BFL) aufweist,
wobei es sich bei der Metalloprotease um MMP1 handelt und die ladungsändernde Peptidsequenz die Sequenz Ac-N-D-G-P-Q-A-I-A-G-Q-G-A-G-Diaminoethyl-BFL aufweist,
wobei es sich bei der Metalloprotease um MMP3 handelt und die ladungsändernde Peptidsequenz die Sequenz Ac-D-G-P-K-P-V-E-Nva-Y-N-K(ε-BFL)-NH₂ aufweist,
wobei es sich bei der Metalloprotease um MMP8 handelt und die ladungsändernde Peptidsequenz die Sequenz gemäß Ac-N-D-G-P-Q-G-Y-A-G-Q-G-A-G-Diaminoethyl-BFL aufweist,
wobei es sich bei dem Cathepsin um Cathepsin-S handelt und die ladungsändernde Peptidsequenz die Sequenz N-SUC-E-G-R-W-H-T-V-G-L-R-W-E-C(Cy5)-R-CO-NH₂ aufweist,
wobei es sich bei dem Cathepsin um Cathepsin-D handelt und die ladungsändernde Peptidsequenz die Sequenz N-SUC-D-D-L-V-V-L-F-V-K-K-C(Cy5)-A-CO-NH₂ aufweist,
wobei es sich bei der bakteriellen Protease um Omp-T handelt und die ladungsändernde Peptidsequenz die Sequenz Ac-SUC-D-G-D-K-Y-R-R-A-W-G-D-T-I-Diaminoethyl-BFL aufweist,
wobei es sich bei der bakteriellen Protease um SspB handelt und die ladungsändernde Peptidsequenz die Sequenz Ac-D-G-D-A-F-S-K-A-L-P-K(ε-BFL)-NH₂ aufweist,
wobei es sich bei dem Kallikrein um Kallikrein 2 handelt und die ladungsändernde Peptidsequenz die Sequenz Ac-N-D-G-D-T-F-R-S-A-A-G-K(ε-BFL)-NH₂ aufweist,
wobei es sich bei dem Kallikrein um Kallikrein 3 handelt und die ladungsändernde Peptidsequenz die Sequenz Ac-N-D-G-S-S-I-Y-Q-S-S-T-G-Diaminoethyl-BFL aufweist oder
wobei es sich bei dem Calpain um Calpain 1 oder Calpain 2 handelt und die ladungsändernde Peptidsequenz die Sequenz N-SUC-E-P-L-F-A-A-R-K(ε-BFL)-NH₂ aufweist.

2. Verfahren nach Anspruch 1, wobei es sich bei dem Fluoreszenz-Tag um einen Fluoreszenzfarbstoff handelt.

3. Verfahren nach Anspruch 1 oder 2, wobei die Spaltprodukte durch Gelelektrophorese, Kapillarelektrophorese oder eine Kombination von DC- und AC-elektrokinetischen Techniken von der komplexen Probe getrennt werden.

4. Verfahren nach einem der Ansprüche 1-3, wobei die proteolytische Signatur durch Nachweisen und Einordnen einzelner Substrate im Peptidgemisch gemäß ihrer Wanderung erzeugt wird.

5. Verfahren nach Anspruch 4, wobei die einzelnen Substrate über das Tag nachgewiesen werden.

6. Verfahren nach einem der Ansprüche 1-5, wobei die proteolytische Signatur mehrere Klassen von Proteaseaktivität umfasst.

7. Verfahren nach Anspruch 6, wobei die mehreren Klassen von Proteaseaktivität aus Serin-, Cystein-, Threonin-, Aspartyl- und Metallopeptidase-Aktivität ausgewählt sind.

8. Verfahren nach Anspruch 6, wobei die mehreren Klassen von Proteaseaktivität durch separates Inkubieren der komplexen Probe mit einem Aktivitätshemmer nachgewiesen werden.

9. Verfahren nach Anspruch 8, wobei der Aktivitätshemmer aus einem Metallchelator, einem Cysteinpeptidase-Hemmer und einem Elastase-spezifischen Hemmer ausgewählt ist.

10. Verfahren nach Anspruch 9, wobei der Aktivitätshemmer aus EDTA, E-64, CAO74 und einem Chlormethylketon-Hemmstoff ausgewählt ist.

## Revendications

1. Procédé d'identification d'une activité de protéase dans un échantillon biologique complexe choisi parmi le sang, le plasma et le sérum, le procédé comprenant :
a) l'incubation de l'échantillon complexe avec une banque de peptides synthétiques pour générer des produits de clivage, la banque de peptides synthétiques comprenant des combinaisons de paires d'acides aminés, des résidus chargés positivement et négativement, ou des résidus modifiés à différentes positions sur la longueur des peptides synthétiques, et une étiquette fluorescente ;
b) la séparation des produits de clivage de l'échantillon complexe ; et
c) la génération d'une signature protéolytique pour l'échantillon complexe ; dans lequel la banque de peptides synthétiques comprend des substrats peptidiques fluorescents à changement de charge synthétiques spécifiques pour des protéases, dans lequel les séquences peptidiques à changement de charge ciblent sélectivement des protéases choisies parmi les protéases liées à la coagulation, les métalloprotéases, les cathepsines, les protéases bactériennes, les kallikréines et les calpaïnes,
dans lequel la protéase apparentée à la coagulation est la thrombine et la séquence peptidique à changement de charge a la séquence Ac-N-D-D-Nle-T-P-R-G-S-A-G-A-G-A-G-diamino-éthyl-Bodipy FL (BFL),
dans lequel la métalloprotéase est MMP1 et la séquence peptidique à changement de charge présente la séquence Ac-N-d-G-P-Q-A-I-A-G-Q-G-A-G-diamino-éthyl-BFL,
dans lequel la métalloprotéase est MMP3 et la séquence peptidique à changement de charge a la séquence Ac-D-G-P-K-P-V-E-Nva-Y-N-K(ε-BFL)-NH₂,
dans lequel la métalloprotéase est MMP8 et la séquence peptidique à changement de charge a la séquence indiquée dans Ac-N-D-G-P-Q-G-Y-A-G-Q-G-A-G-diamino-éthyl-BFL, dans lequel la cathepsine est la cathepsine-S et la séquence peptidique à changement de charge a la séquence N-SUC-E-G-R-W-H-T-V-G-L-R-W-E-C(Cy5)-R-CO-NH₂,
dans lequel la cathepsine est la cathepsine-D et la séquence peptidique à changement de charge a la séquence N-SUC-D-D-L-V-V-L-F-V-K-K-C(Cy5)-A-CO-NH₂,
dans lequel la protéase bactérienne est Omp-T et la séquence peptidique à changement de charge a la séquence Ac-SUC-D-G-D-K-Y-R-R-A-W-G-D-T-I-diamino-éthyl-BFL, dans lequel la protéase bactérienne est SspB et la séquence peptidique à changement de charge a la séquence Ac-D-G-D-A-F-S-K-A-L-P-K(ε-BFL)-NH₂,
dans lequel la kallicréine est la kallicréine 2 et la séquence peptidique à changement de charge a la séquence Ac-N-D-G-D-T-F-R-S-A-A-G-K(ε-BFL)-NH₂,
dans lequel la kallicréine est la kallicréine 3 et la séquence peptidique à changement de charge a la séquence Ac-N-D-G-S-S-I-Y-Q-S-S-T-G-diamino-éthyl-BFL, ou
dans lequel la calpaïne est la calpaïne 1 ou la calpaïne 2 et la séquence peptidique à changement de charge a la séquence N-SUC-E-P-L-F-A-A-R-K(ε-BFL)-NH₂.

2. Procédé selon la revendication 1, dans lequel l'étiquette fluorescente est un colorant fluorescent.

3. Procédé selon la revendication 1 ou 2, dans lequel les produits de clivage sont séparés de l'échantillon complexe par électrophorèse sur gel, électrophorèse capillaire ou une combinaison de techniques électrocinétiques DC et AC.

4. Procédé selon l'une quelconque des revendications 1 à 3, dans lequel la signature protéolytique est générée en détectant et en classant des substrats individuels dans le mélange peptidique selon la migration.

5. Procédé selon la revendication 4, dans lequel les substrats individuels sont détectés par l'intermédiaire de l'étiquette.

6. Procédé selon l'une quelconque des revendications 1 à 5, dans lequel la signature protéolytique comprend plusieurs classes d'activité de protéase.

7. Procédé selon la revendication 6, dans lequel les plusieurs classes d'activité de protéase sont choisies parmi une activité de sérine, cystéine, thréonine, aspartyle et métallopeptidase.

8. Procédé selon la revendication 6, dans lequel les plusieurs classes d'activité de protéase sont détectées en incubant séparément l'échantillon complexe avec un inhibiteur d'activité.

9. Procédé selon la revendication 8, dans lequel l'inhibiteur d'activité est choisi parmi un chélateur de métal, un inhibiteur de cystéine peptidase et un inhibiteur spécifique d'élastase.

10. Procédé selon la revendication 9, l'inhibiteur d'activité étant choisi parmi EDTA, E-64, CAO74, et un inhibiteur de chlorométhylcétone.
